# FASCICULE DE BREVET EUROPEEN

(11) **EP 2 654 712 B1**
(45) Date de publication et mention de la délivrance du brevet: **22.02.2017**
(21) Numéro de dépôt: 11813412.1
(22) Date de dépôt: 20.12.2011
(51) Int. Cl.: A61K 8/73, A61K 8/81, A61Q 19/00, A61N 5/06

(54) **COMPOSITION LIQUIDE ET STÉRILE POUR LE COMBLEMENT DES RIDES**
STERILE FLÜSSIGZUSAMMENSETZUNG ZUM AUFFÜLLEN VON FALTEN
STERILE LIQUID COMPOSITION FOR FILLING WRINKLES

(30) Priorité: 20.12.2010 FR 1060788; 11.02.2011 US 201161441757 P
(43) Date de publication de la demande: 30.10.2013
(73) Titulaire: L'Oréal, 75008 Paris (FR)
(72) Inventeur: SAMAIN, Henri, F-91570 Bievres (FR); SIMONNET, Jean-Thierry, F-Cachan 94230 (FR)
(74) Mandataire: Nony
(86) Numéro de dépôt international: PCT/IB2011/055806
(87) Numéro de publication internationale: WO 2012/085835

(56) Documents cités:
- WO-A1-2009/018555
- WO-A2-2007/106457
- MARTENS P ET AL: "Characterization of hydrogels formed from acrylate modified poly(vinyl alcohol) macromers", POLYMER, ELSEVIER SCIENCE PUBLISHERS B.V, GB, vol. 41, no. 21, 1 octobre 2000 (2000-10-01), pages 7715-7722, XP004201762, ISSN: 0032-3861, DOI: 10.1016/S0032-3861(00)00123-3
- NGUYEN K T ET AL: "Photopolymerizable hydrogels for tissue engineering applications", BIOMATERIALS, ELSEVIER SCIENCE PUBLISHERS BV., BARKING, GB, vol. 23, no. 22, 1 novembre 2002 (2002-11-01), pages 4307-4314, XP004374369, ISSN: 0142-9612, DOI: 10.1016/S0142-9612(02)00175-8
- ELISSEEFF J ET AL: "TRANSDERMAL PHOTOPOLYMERIZATION FOR MINIMALLY INVASIVE IMPLANTATION", PROCEEDINGS OF THE NATIONAL ACADEMY OF SCIENCES, NATIONAL ACADEMY OF SCIENCES, WASHINGTON, DC; US, vol. 96, no. 6, 16 mars 1999 (1999-03-16), pages 3104-3107, XP001009855, ISSN: 0027-8424, DOI: 10.1073/PNAS.96.6.3104
- K.ICHIMURA AND S.WATANABE: "Preparation and characteristics of photocrosslinkable poly(vinyl alcohol)", JOURNAL OF POLYMER SCIENCE, vol. 20, 1982, pages 1419-1432, XP002658584,

## Description

La présente invention concerne le traitement des défauts et imperfections esthétiques de la peau et des lèvres.

Ces imperfections sont d'origines diverses et peuvent êtres dues ou non à l'âge.

Ainsi, des imperfections de la peau peuvent résulter d'infections cutanées, comme par exemple les cicatrices de l'acné, ou des effets d'intervention chirurgicale comme des cicatrices.

En ce qui concerne les imperfections liées à l'âge, les rides en sont manifestement les plus représentatives.

Parmi ces rides, on peut distinguer les rides « marquées », très visibles et pouvant présenter une profondeur de 300 microns ou plus, les rides « moyennes », visibles et de profondeur variant entre 150 microns et 300 microns et les rides « légères », moins visibles que les précédentes et de profondeur inférieure à 150 microns.

Pour des raisons évidentes, la présence de rides sur le visage pose souvent des problèmes d'esthétique qui, selon les personnes, peuvent être difficiles à vivre et il existe donc un besoin de palliatif(s) pour contrer la visibilité des imperfections précitées et plus particulièrement les rides.

Il est connu que ces imperfections peuvent être corrigées par l'application sur la peau d'actifs susceptibles de tendre la peau par effet tenseur, cette tension permettant de lisser la peau et de faire diminuer, voire disparaître, de façon immédiate les rides et les ridules.

Toutefois, appliquées topiquement, ces actifs ne pénètrent que très faiblement, n'exerçant ainsi qu'un effet limité dans le temps et en intensité. Qui plus est, ils ne sont efficaces que sur les rides superficielles du visage, et sont d'efficacité moindre sur celles plus profondes pour lesquelles leur action est en revanche faible et très peu durable.

Une autre solution consiste à traiter les muscles sous cutanés, appelés muscles peaussiers, par exemple par une intervention chirurgicale.

Ainsi, les rides formées autour des yeux, du front et de la bouche peuvent être traitées via les muscles peaussiers correspondants. Même si cette approche présente l'avantage de durer dans le temps, elle présente plusieurs inconvénients qui réduisent beaucoup son développement. Elle est douloureuse, peut être contre-indiquée et relativement coûteuse. Elle est en outre réputée pour présenter le risque de provoquer des effets esthétiques indésirables comme notamment, un manque d'aspect naturel, voire des effets de relâchement de la peau ou de paralysie locale.

Une autre alternative de traitement vise à traiter les rides par injection d'actifs de comblement. Cette technique permet d'améliorer l'état de surface en aplanissant la peau et en réduisant les reliefs. Elle est également utilisée pour augmenter le volume de certaines parties du corps par exemple du visage, notamment pour le bord des lèvres mais aussi pour réparer des déformations dues par exemple à un accident.

Dans ce type d'injection, on utilise des molécules telles que des protéines, en particulier le collagène, des dérivés de sucres, tels que l'acide hyaluronique. Ces composés présentent une viscosité suffisante pour conférer au site d'injection un effet volume permettant de diminuer significativement voire d'altérer totalement la visibilité de la dépression d'origine qui figurait initialement et qualifiée de ride.

Aujourd'hui, l'injection la plus courante est réalisée avec un gel d'acide hyaluronique mis en oeuvre sous une forme réticulée, qui s'est révélée beaucoup plus efficace que l'acide hyaluronique non réticulé. Pour cela, l'acide hyaluronique présente lors de l'emploi un niveau de réticulation destiné à lui donner la consistance recherchée.

Toutefois, cette technique présente également des limitations.

En particulier, la viscosité inhérente à la réticulation, et nécessaire au comblement de la ride, génère malheureusement à l'injection, une sensation douloureuse pour le patient.

Une alternative serait donc de procéder à la réticulation et/ou polymérisation seulement *in situ,* et d'injecter l'agent de comblement sous une forme non réticulée et donc doté d'une viscosité significativement réduite.

Toutefois, l'utilisation de composés fluides aptes à s'épaissir une fois injecté par effet de réticulation se heurte dans de nombreux cas à plusieurs problèmes. En effet, en général, les polymères chimiques réticulables requièrent l'emploi d'oxydants puissants (peroxyde), de catalyseur métallique, et/ou une élévation de la température, en milieu anhydre, qui sont des conditions peu compatibles avec un milieu biologique.

Ainsi, les monomères, comme les dérivés cyanoacrylates, nécessitent pour leur polymérisation la présence de composés additionnels peu compatibles avec une utilisation cutanée. De même, les composés aptes à réticuler sous l'effet de la lumière nécessitent pour leur part en général des photoinitiateurs incompatibles avec la physiologie et impliquent souvent des irradiations lumineuses importantes, en particulier dans le domaine des ultraviolets.

A titre illustratif des techniques déjà proposées et reposant sur une réticulation *in situ* de polymère, peuvent notamment être citées l'utilisation d'un composé photoréticulable pour le traitement des cheveux (WO 2004/054527) et des compositions injectables comprenant un monomère acrylate hydrogel polymérisable et un initiateur qui peut être un photoinitiateur, un initiateur thermique ou chimique telles que proposées dans WO 2007/106457 pour créer une augmentation de tissu biologique.

On connaît également de EP 0713859, des dérivés d'acide cinnamique photodimérisables et en particulier des dérivés photoréticulables d'acide hyaluronique fonctionnalisés avec des dérivés d'acide cinnamique. Toutefois, ces derniers n'y sont décrits qu'à des fins de préparation de pellicules photodurcissables.

Il subsiste donc un besoin pour de nouvelles compositions et méthodes pour masquer les reliefs marqués, notamment les rides « marquées » et les rides « moyennes », et résolvant en tout ou partie les problèmes discutés ci-dessus.

C'est en particulier un objet de l'invention que de proposer une composition et un dispositif qui permettent de traiter toutes sortes de rides, allant des rides légères aux rides marquées ainsi que les autres reliefs de la peau tels que les vergetures, cicatrices, plis et pores dilatés.

De manière inattendue, les inventeurs ont constaté qu'il s'avère possible de répondre à ce besoin sous réserve de mettre en oeuvre des composés spécifiques.

Selon un premier aspect, l'invention a pour objet une composition liquide et stérile, dédiée à une administration dans ou à travers la peau et/ou les lèvres comprenant dans un milieu physiologiquement acceptable au moins un composé photoréticulable, ledit composé comportant au moins un groupe photodimérisable activé possédant au moins une double liaison activée et choisi parmi :
a) les groupes photodimérisables portant une fonction stylbazolium de formule (Ia) ou (Ib) : où 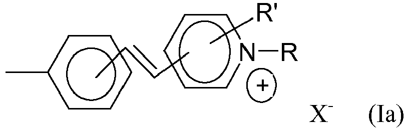
   - R représente l'atome d'hydrogène, un groupement alkyle en C₁-C₄ ou hydroxyalkyle en C₁-C₄.
   - R' représente l'atome d'hydrogène ou un groupement alkyle en C₁-C₄, et
   - X⁻ désigne un ion choisi parmi les ions chlorures, bromures, iodures, perchlorates, tetrafluoroborates, méthylsulfate, phosphates, sulfates, méthanesulfonates, p-toluènesulfonate. 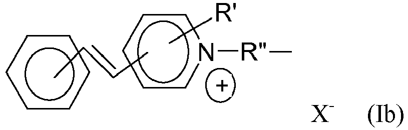
   où
   - R" désigne un radical alkylène divalent ayant de 2 à 8 atomes de carbone,
   - R' représente l'atome d'hydrogène ou un groupement alkyle en C₁-C₄, et
   - X⁻ ayant la même signification que celle décrite pour la formule (Ia) précédente.
b) les groupes photodimérisables portant une fonction styrylazolium de formule (II) : 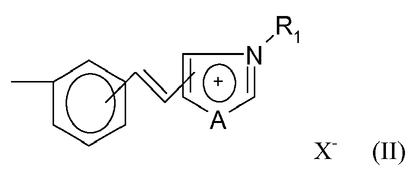 dans laquelle :
   - R₁ désigne l'atome d'hydrogène, un groupement alkyle en C₁-C₄ ou hydroxyalkyle en C₁-C₄,
   - A désigne un atome de soufre, un atome d'oxygène, ou un groupement NR' ou C(R')₂, R', avec R' représentant l'atome d'hydrogène ou un groupement alkyle en C₁-C₄, et
   - X⁻ ayant la même signification que celle décrite pour la formule (Ia) précédente.
le ou les groupe(s) photodimérisable(s) étant portés par un polymère de type polyvinylacétate partiellement ou totalement hydrolysé, un polysaccharide ou un alcool polyvinylique.

Par "milieu physiologiquement acceptable", on entend un milieu dénué de toxicité et compatible avec l'injection et/ou l'application de la composition dans ou à travers la peau et/ou les lèvres.

Par « stérile », on entend qualifier un environnement apte à garantir au composé et/ou à la composition qui le contient l'innocuité requise pour une administration dans ou à travers la peau et/ou les lèvres, notamment intraépidermique et/ou intradermique et/ou sous-cutanée. En particulier, il est essentiel que la composition formée du milieu physiologiquement acceptable contenant ledit composé photoréticulable et devant être administrée selon une technique d'injection, par exemple selon la technique de mésothérapie, soit dénuée de tout corps contaminant susceptible d'initier une réaction secondaire indésirable au niveau de l'organisme hôte.

Au sens de la présente invention, un groupement photodimérisable est un groupement chimique conduisant à des réactions de photodimérisation sous irradiation.

Par photodimérisation, on entend au sens de la présente invention, une réaction chimique entre deux doubles liaisons (de type 2 + 2) ou de deux paires de doubles liaisons (de type 4 + 4) et plus préférentiellement entre deux doubles liaisons (de type 2 + 2).

Ainsi, la double liaison considérée selon l'invention, lorsqu'elle est photostimulée ou en d'autres termes soumise à un rayonnement spécifique, généralement UV, s'avère apte à réagir avec une autre double liaison par cyclisation.

Comme détaillée ci-après, les doubles liaisons interagissant sont des fonctions éthyléniques, c'est-à-dire de type CH₂=CH₂.

Au sens de l'invention, la double liaison considérée est dite activée. Cette caractérisation signifie que la double liaison est photodimérisable spontanément en réponse à un photostimuli, sans requérir la présence obligatoire d'un photoinitiateur ou d'un initiateur chimique.

Par conséquent, une composition selon l'invention est avantageusement dénuée de photoinitiateur et/ou d'initiateur chimique.

L'activation de la double liaison destinée à se dimériser est communément induite par la présence à proximité de celle-ci, généralement en position alpha, d'un groupe électroattracteur, tel que par exemple un noyau aromatique à l'image d'un phényle.

En ce qui concerne le composé photoréticulable, il comprend au moins un groupe photodimérisable tel que défini précédemment.

Le composé photoréticulable dérive de la fonctionnalisation d'un squelette naturel, de préférence polymérique, par au moins un groupe photodimérisable.

Cette fonctionnalisation relève des compétences de l'homme de l'art.

Comme précisé précédemment, ces squelettes polymériques sont choisis parmi les polymères naturels tels que les polysaccharides et les polymères synthétiques tels que le polyvinylacétate partiellement ou totalement hydrolysé et l'alcool polyvinylique (PVA).

Les polysaccharides convenant à l'invention peuvent notamment être choisis parmi le sulfate de chondroïtine, le kératane, le sulfate de kératane, l'héparine, le sulfate d'héparine, le xanthane, la carraghénane, le chitosane, la cellulose et ses dérivés, l'alginate, l'amidon, le dextrane, le pullulane, le galactomannane et leurs sels biologiquement acceptables.

Selon un autre de ses aspects, l'invention a pour objet un système de traitement de la peau et/ou des lèvres comportant :
- un conditionnement contenant au moins une dose d'une composition telle que définie ci-dessus et,
- un dispositif d'injection dans ou à travers la peau et/ou les lèvres ou un dispositif de microperforation de la peau et/ou des lèvres dédié à l'administration de ladite dose.

Par « zone de traitement » on entend au sens de la présente invention une zone de la peau et/ou des lèvres ayant reçue une partie de la composition et/ou de la lumière destinée à provoquer la réticulation.

Par « composition injectable selon l'invention » on entend aussi bien une composition revendiquée pour son usage thérapeutique que la composition d'un dispositif de traitement selon l'invention.

### Composé photoréticulable

Selon l'invention, un composé photoréticulable comprend au moins un motif photodimérisable doté d'une double liaison activée photodimérisable, porté par un polymère de type polyvinylacétate partiellement ou totalement hydrolysé, un polysaccharide ou un alcool polyvinylique.

Avantageusement, le composé photoréticulable selon l'invention peut porter une ou plusieurs fonction(s) cyclisable(s) selon une réaction 2/2, sensible(s) à l'irradiation lumineuse, en l'absence de photoinitiateur et/ou d'initiateur chimique.

Un composé réticulable selon l'invention peut être pré-réticulé.

Toutefois, pour des raisons évidentes, ce degré de réticulation est ajusté pour ne pas conférer une viscosité trop élevée au gel correspondant et qui serait susceptible de générer un inconfort voire une douleur au moment de l'administration et dont la présente invention cherche précisément à s'affranchir.

### Groupement photodimérisable

Par groupement photodimérisable, on entend, au sens de la présente invention, un groupement chimique conduisant à des réactions de photodimérisation sous irradiation.

Par photodimérisation, on entend, au sens de la présente invention une réaction chimique entre deux doubles liaisons (de type 2 + 2) ou deux paires de doubles liaisons (de type 4 + 4).

Le cas d'une réaction entre deux doubles liaisons peut se schématiser de la manière suivante :

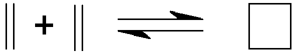

Ces réactions de photodimérisation sont définies dans le document Advanced Organic Chemistry, J Marck, 4th édition, Wiley Interscience, NY 1992, p 855.

Les matériaux possédant des groupements photodimérisables selon l'invention présentent l'avantage d'être stables vis à vis de l'oxygène, de l'humidité, et de la chaleur, et de conduire à une réticulation réversible.

En outre, les groupements photodimérisables selon l'invention sont très photosensibles. Par conséquent, une irradiation même de faible énergie conduit à une réticulation rapide et efficace du matériau, ce qui, dans le cas d'une application en cosmétique, induit une irradiation de courte durée et de faible énergie qui n'entraîne pas de dégradation de la peau et/ou des lèvres.

Les groupements photodimérisables utilisables selon l'invention sont choisis parmi :
a) les groupes photodimérisables portant une fonction stylbazolium de formule (Ia) ou (Ib) : 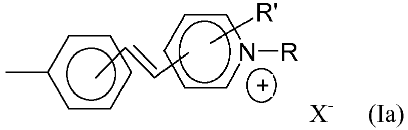 où
   - R représente l'atome d'hydrogène, un groupement alkyle en C₁-C₄ ou hydroxyalkyle en C₁-C₄, de préférence, R représente l'atome d'hydrogène, un groupe méthyle, un groupe éthyle ou un hydroxyéthyle, préférentiellement, R est un groupe méthyle ;
   - R' représente l'atome d'hydrogène ou un groupement alkyle en C₁-C₄, de préférence, R' représente l'atome d'hydrogène ; et
   - X⁻ désigne un ion choisi parmi les ions chlorures, bromures, iodures, perchlorates, tetrafluoroborates, méthylsulfate, phosphates, sulfates, méthanesulfonates, p-toluènesulfonate, de préférence, X⁷ est un ion choisi parmi les ions chlorure, méthylsulfates, préférentiellement, X⁻ est l'ion méthylsulfate. 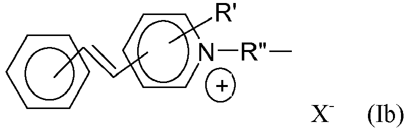
   où
   - R" désigne un radical alkylène (divalent) ayant de 2 à 8 atomes de carbone, de préférence, R" désigne un radical alkylène (divalent) ayant de 2 à 4 atomes de carbone ;
   - R' représente l'atome d'hydrogène ou un groupement alkyle en C₁-C₄, avec de préférence R' représentant l'atome d'hydrogène ; et
   - X⁻ ayant la même signification que celle décrite pour la formule (Ia) précédente.
b) les groupes photodimérisables portant une fonction styrylazolium de formule (II) : 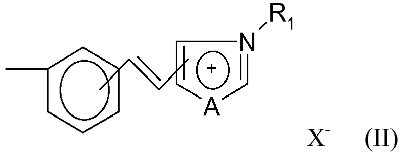 dans laquelle :
   - R₁ désigne l'atome d'hydrogène, un groupement alkyle en C₁-C₄ ou hydroxyalkyle en C₁-C₄ ;
   - A désigne un atome de soufre, un atome d'oxygène, ou un groupement NR' ou C(R')₂, R' ; avec R' représentant l'atome d'hydrogène ou un groupement alkyle en C₁-C₄, R' représentant de préférence l'atome d'hydrogène ; et
   - X⁻ ayant la même signification que celle décrite pour la formule (Ia) précédente.

De tels groupements chimiques possèdent des doubles liaisons activées, de sorte que la photodimérisation de ces doubles liaisons se déclenche spontanément dans le domaine de l'UVA, sans nécessiter de photoinitiateur.

Selon un mode de réalisation préférée, les groupements photodimérisables utilisables selon l'invention portent une fonction stylbazolium de formule (Ia) : 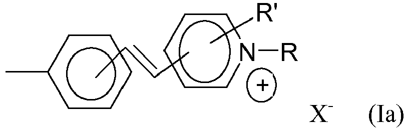 où
- R représente l'atome d'hydrogène, un groupement alkyle en C₁-C₄ ou hydroxyalkyle en C₁-C₄.
- R' représente l'atome d'hydrogène ou un groupement alkyle en C₁-C₄, et
- X⁻ désigne un ion choisi parmi les ions chlorures, bromures, iodures, perchlorates, tetrafluoroborates, méthylsulfate, phosphates, sulfates, méthanesulfonates, p-toluènesulfonate.
- En ce qui concerne les composés ayant des groupes photodimérisables portant une fonction stylbazolium, ils sont obtenus par réaction du polymère considéré avec une entité chimique comportant un groupe de formule (Ia) ou (Ib).

De préférence, l'entité chimique comportant un groupe (Ia) porte un groupe réactif W de type aldéhyde ou acétal.

En d'autres termes, cette entité chimique répond à la formule générale W-A, avec A désignant le groupe (Ia).

Comme entités chimiques pouvant être utilisées pour greffer des groupements de type styrylpyridinium, on peut notamment citer les sels quaternaires de 2-(4-formylstyryl)-pyridinium, 4-(4-formyl-styryl)-pyridinium, 2-(3-formylstyryl)-pyridinium, N-methyl-2-(4-formylstyryl)pyridinium, N-methyl-3-(4-formylstyryl)-pyridinium, N-methyl-2-(3-formylstyryl)-pyridinium, N-methyl-2-(2-formylstyryl)pyridinium, N-ethyl-2-(4-formyl-styryl)-pyridinium, N-(2-hydroxyethyl)-2-(4-formylstyryl)-pyridinium, N-(2-hydroxyethyl)-4-(4-formylstyryl)-pyridinium, N-methyl-4-(4-formylstyryl)-pyridinium, N-methyl-4-(3-formylstyryl)-pyridinium.

Les sels quaternaires de pyridinium peuvent être des sels chlorures, bromures, iodures, perchlorates, tetrafluoroborates, méthosulfate, phosphates, sulfates, méthanesulfonates, p-toluènesulfonate. De telles entités chimiques sont décrites dans GB-A-2030575.

Comme exemple d'entités, on peut citer le 4-(4-formylphényléthényl)1-méthylpyridiunium methosulfate, le 1-(3-ethoxycarbonylmethyl)-4-[2-(4-formylphenyl)ethenyl]pyridinium bromide, le 1-(methoxycarbonylpropyl)-4-[2-(4-formylphenyl)ethenyl]pyridinium bromide. De telles entités sont décrites dans US 2007/0112094.

De préférence, on utilise le n-méthyl-4-(4-formylstyryl)pyridinium methylsulfate (RN= 74401-04-0), notamment commercialisé par la société WAKO.

Avantageusement, les entités chimiques de formule W-A réagissent avec un polymère de type alcool polyvinylique ou polyvinyl acétal comme décrit dans les documents cités précédemment.

Par exemple, un polymère greffé polyvinyl alcool comportant des motifs de structure suivante est ainsi obtenu :

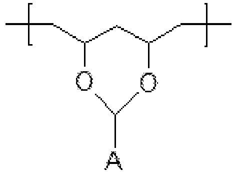

Des polymères alcool polyvinyliques greffés de groupement stryrylpyridinium sont notamment décrits dans la publication Ichimura K et al, Préparation and Characteristics of photocross-linkable poly(vinyl alcohol), Journal of polymer science, polymer chemistry édition, Vol 20, 1419-1432 (1982).

Les polymères peuvent être obtenus par réaction d'alcool polyvinylique ou polyvinylacétate partiellement hydrolysé avec des sels de styrylpyridinium à groupe formyl ou acétal tels que décrits dans GB-A-2030575, WO 96/29312, US 5061603, GB-A-2076826, EP-A-092901.

Des polymères de cellulose greffés de groupements styrylpyrydinium sont notamment décrits dans US 2007/0112094.

De préférence, l'entité chimique comportant un groupe (Ia) porte un groupe réactif qui est un atome de chlore.

Dans cette variante, l'entité chimique répond à une formule générale Cl-A', avec A' désignant le groupe (Ia).

Comme entité chimique de type Cl-A', on utilise de préférence celle de formule : 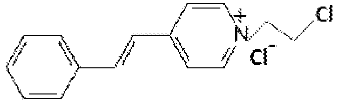 correspondant au composé chloré (1), décrit dans les exemples de préparations ci-après.

Avantageusement, le composé photoréticulable comprenant les groupes (Ib) est avantageusement obtenu par réaction de l'entité Cl-A' avec le polysaccharide choisi parmi ceux définis précédemment, en présence d'eau.
- Pour leur part, les composés ayant des groupes photodimérisables portant une fonction styrylazolium sont obtenus par réaction du polymère avec une entité chimique comportant un groupe de formule (II).

De préférence, l'entité chimique comportant un groupe (II) porte un groupe réactif W de type aldéhyde ou acétal.

En d'autres termes, l'entité chimique répond à une formule générale W-B, avec B désignant le groupe (II).

Comme entités chimiques pouvant être utilisées pour greffer des groupements de type styrylazolium, on peut citer celles décrites dans EP-A-313220.

Avantageusement, ces entités chimiques de formule W-B réagissent avec un polymère de type alcool polyvinylique ou polyvinyl acétal comme décrit dans les documents cités précédemment.

Un polymère greffé polyvinyl alcool comportant des motifs de structure suivante est ainsi obtenu :

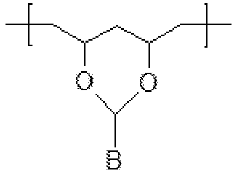

Des polymères alcool polyvinylique greffés de groupement styrylazolium sont notamment décrits dans EP-A-313220. Dans ce document, ces polymères peuvent être obtenus par réaction d'alcool polyvinylique ou polyvinylacétate partiellement hydrolysé avec des sels de styrylazolium à groupe aldéhyde ou acétal.

Avantageusement, dans les compositions de l'invention, le composé comportant au moins un groupement photodimérisable est un polymère hydrocarboné choisi parmi l'alcool polyvinylique, le polyvinylacétate totalement ou partiellement hydrolysé et les polysaccharides choisis parmi le sulfate de chondroïtine, le kératane, le sulfate de kératane, l'héparine, le sulfate d'héparine, le xanthane, la carraghénane, le chitosane, la cellulose et ses dérivés, l'alginate, l'amidon, le dextrane, le pullulane, le galactomannane et leurs sels biologiquement acceptables.

Plus avantageusement encore, il se présente sous la forme de particules, en particulier de particules dispersées.

Ainsi, dans ce dernier cas, les particules de polymère sont très préférentiellement des particules d'alcool polyvinylique.

Ainsi, selon une variante de réalisation, le composé photoréticulable est un alcool polyvinylique (PVA) fonctionnalisé en partie par une ou plusieurs fonction(s) hydroxyle(s) et une ou plusieurs fonction(s) de formule (III) :

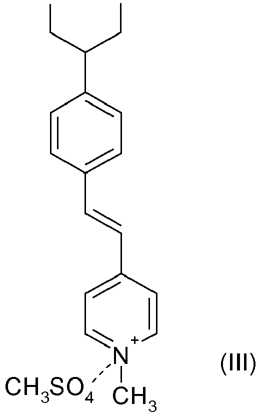

Le degré de polymérisation de l'alcool polyvinylique peut être compris entre 100 et 5000.

Avantageusement, le polymère a un taux de substitution, en % de fonctions de formule (Ia), (Ib) ou (II) telle que définie ci-dessus, pouvant être compris entre 0.1 et 25.

Le schéma suivant représente une variante où le polymère est le PVA-SbQ (polymère de type alcool polyvinylique PVA portant quelques fonctions hydrolysées et quelques fonctions greffées par des entités stylbazoliums), qui est apte à réticuler sous l'effet de la lumière, comme illustré ci-après.

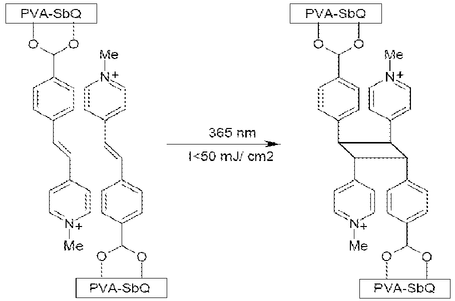

Ces matériaux sont particulièrement appréciés car ils ne nécessitent pas de photoinitiateur et réagissent à une lumière visible ou à un rayonnement pouvant comporter à la fois une lumière UV et une lumière visible, en particulier une faible dose d'UV.

Il est à noter que le choix de la lumière UV et/ou visible est généralement ajusté en considérant la profondeur à laquelle une composition selon l'invention est injectée au niveau de l'épiderme à traiter.

Ainsi, si la composition est injectée au dessus de 1000 µm, on privilégie les matériaux réagissant à un rayonnement comportant une lumière visible, en particulier entre 500 nm et 1000 nm.

Si la composition est injectée entre 300 et 1000 µm, les matériaux réagissant à un rayonnement comportant une lumière visible, en particulier entre 450 nm et 650 nm ou comportant à la fois une lumière UV et une lumière visible sont en revanche privilégiés.

Enfin, si la composition est injectée entre 0 et 300 µm, on privilégie les matériaux réagissant à un rayonnement comportant une lumière UV ou comportant à la fois une lumière UV et une lumière visible dans un rapport 50% dans la zone 350-400 nm et 50% dans la zone 400-500 nm ou à une lumière visible.

L'invention vise également, comme polymère fonctionnalisé, des dispersions aqueuses d'un polymère de type polyvinylacétate partiellement saponifié (hydrolysé) et portant des groupes stylbazoliums, mis en présence, notamment mélangé, avec des particules de polyvinylacétate.

Selon une autre variante de réalisation, le composé photoréticulable est figuré par un polysaccharide qui est fonctionnalisé par des groupements photodimérisables.

Il peut notamment s'agir d'un polysaccharide pouvant notamment être choisis parmi le sulfate de chondroïtine, le kératane, le sulfate de kératane, l'héparine, le sulfate d'héparine, le xanthane, la carraghénane, le chitosane, la cellulose et ses dérivés, l'alginate, l'amidon, le dextrane, le pullulane, le galactomannane et leurs sels biologiquement acceptables.

Le degré de fonctionnalisation est bien entendu ajusté pour pouvoir conférer le degré de réticulation requis lors de l'activation *in situ.*

Selon l'invention, le degré de fonctionnalisation en motif photodimérisable est au moins de 0,1 %, voire au moins de 0,5 %, voire au moins de 2%.

Comme précisé précédemment, dans une composition selon l'invention, les groupes photodimérisables sont portés par un polymère de type polyvinylacétate, un alcool polyvinylique ou un polysaccharide.

De préférence, dans une composition selon l'invention, les groupes photodimérisables sont portés par un alcool polyvinylique.

Le composé réticulable peut être véhiculé dans un milieu physiologiquement acceptable et en particulier un milieu aqueux voire de l'eau pure.

La présence d'eau ne l'empêche pas de photoréticuler, ce qui présente l'avantage de permettre d'effectuer une photoréticulation *in situ.*

Selon l'invention, le composé photoréticulable possède un poids moléculaire moyen en nombre allant de 2000 à 100 000, et de préférence allant de 2000 à 20 000.

### Photodimérisation

La composition peut contenir un unique polymère portant des fonctions de nature différentes ou non.

On peut aussi utiliser un mélange de polymères ayant des fonctions différentes.

En conséquence, les réactions peuvent s'effectuer entre deux groupements photodimérisables de même nature chimique ou non.

Les doubles liaisons activées peuvent réagir sur une autre double liaison de même nature chimique ou réagir avec une autre double liaison de nature chimique différente.

Le protocole de photodimérisation est décrit de manière plus détaillé ci-après.

Le composé réticulable peut être soluble ou dispersé dans la composition mise en oeuvre pour son administration.

### Composition stérile et liquide, injectable

La composition peut être cosmétique ou non.

Comme précisé précédemment, les compositions considérées selon l'invention se doivent d'être administrées sous une forme dénuée de germes viables ou revivifiables, potentiellement infectieux, microbiens connus de l'homme de l'art. Pour satisfaire à cette exigence, le milieu physiologiquement acceptable liquide et le composé photoréticulable formant la composition administrable selon l'invention peuvent être soumis, de manière réunie ou séparée, à un processus de stérilisation selon une des méthodes conventionnelles bien connues de l'homme de l'art, telles que par exemple chauffage en autoclave, irradiations ionisantes, tels que les faisceaux d'électrons et rayons gamma.

La composition peut comporter un milieu aqueux ou non aqueux physiologiquement acceptable.

La composition peut comporter un solvant ou un mélange de solvants physiologiquement acceptables.

A titre de milieu aqueux ou non aqueux convenant à l'invention, on peut par exemple mentionner l'eau, les alcools, des polyols, les éthers de polyols, et des mélanges de ceux-ci. A titre d'agents isotoniques convenant à la préparation d'une composition convenant à l'invention, il peut être mentionné les sucres et le chlorure de sodium.

Les alcools peuvent être choisis parmi les alcanols inférieurs en C₁-C₆, et de préférence choisis parmi l'éthanol, le propanol et l'isopropanol.

Les polyols peuvent être choisis parmi le glycérol, le propylèneglycol, le polyéthylèneglycol, l'hexylèneglycol, la glycérine, et le pentanediol.

Ces polyols auront pour effet de plastifier le polymère réticulé et de modifier ses propriétés mécaniques finales. Le ratio polymère / polyol va de 100/0 à 1/10.

Ainsi, une composition selon l'invention est généralement mise en oeuvre sous la forme d'une solution isotonique stérile aqueuse ou non aqueuse, sous forme de dispersion, de suspension ou d'émulsion.

Le milieu aqueux ou non aqueux peut représenter de 0,1 % à 99 % en poids par rapport au poids total de la composition, de préférence de 30 % à 99 % en poids, voire de 50 % à 99 % en poids.

Le composé photoréticulable peut représenter au moins 2 %, voire au moins 5 %, voire 10% du volume total de la composition.

Le composé photoréticulable peut être présent dans la composition en une teneur allant de 1 à 15 % en poids, par rapport au poids total de la composition, de préférence allant de 5 à 10 % en poids.

Pour des raisons manifestes, la composition est suffisamment fluide pour faciliter l'injection avec une pression modérée.

La composition a par exemple au moment de l'injection une viscosité inférieure à 100.000 cps, et de préférence intérieure à 10.000 cps mesurée à T = 20 °C en CPS.

Une composition selon l'invention peut comporter outre le composé photoréticulable tout excipient habituellement utilisé dans le domaine des solutions stériles injectables.

Avantageusement, une composition selon l'invention est dénuée de photoinitiateur.

A titre représentatifs des photoinitiateurs conventionnels et qui sont donc non requis selon l'invention, peuvent notamment être cités la thioxanthone, le rose de Bengale, la phloxine, l'éosine, l'érythrosine, la fluorescéine, l'acriflavine, la thionine, la riboflavine, la proflavine, les chlorophylles, l'hématoporphyrine, le bleu de méthylène et leurs mélanges, cette liste n'étant pas limitative.

En revanche, une composition selon l'invention peut avantageusement comprendre un traceur optique ou un composé fluorescent.

### Traceur optique / Composé fluorescent

Un tel composé est avantageux dans la mesure où il peut permettre au praticien procédant à l'injection du composé photoréticulable considéré selon l'invention, de contrôler efficacement la localisation du composé injecté et/ou la dose de composé administré.

Cette option lui permet plus facilement d'irradier toute la zone où est la composition.

Par « traceur optique », on entend un composé qui change de couleur ou devient incolore au moment de l'illumination, c'est-à-dire lorsque la polymérisation a lieu, par exemple un composé « fluorescent » qui absorbe la lumière du spectre ultraviolet et éventuellement du visible et qui transforme l'énergie absorbée en lumière fluorescente de plus grande longueur d'onde, émise dans la partie ultraviolet ou visible du spectre.

Il peut s'agit d'azurants optiques, qui peuvent être transparents et incolores, n'absorbant pas dans la lumière visible mais uniquement dans les UV et transformant l'énergie absorbée en lumière fluorescente de plus grande longueur d'onde, par exemple plus longue de 20 nm, mieux 50 nm, voire 100 nm, émise dans la partie visible du spectre.

L'impression de couleur générée par ces azurants peut alors uniquement être engendrée par la lumière purement fluorescente à prédominante bleue, de longueurs d'onde allant de 400 à 500 nm.

Ces composés peuvent être en solution ou particulaires.

Le composé fluorescent peut être un dicétopyrrolopyrrole de formule : 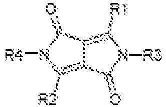 dans laquelle R₁, R₂, R₃ et R₄ indépendamment les uns des autres représentent un atome d'hydrogène ; un atome d'halogène ; un groupement aryle en C₆-C₃₀ ; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement alcoxy(C₁-C₆)carbonyle ; un groupement carboxyalcoxy en C₁-C₆, un groupement pipéridinosulfonyle ; un groupement pyrrolidino ; un groupement alkyle(C₁-C₆)halogénoalkyle(C₁-C₆)amino ; un groupement benzoylalkyle(C₁-C₆) ; un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C₁-C₆, un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène, ce radical alkyle pouvant être interrompu par un hétéroatome.

Le composé fluorescent peut être un naphtalimide, de formule : 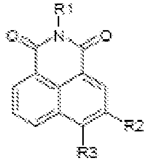 où
R₁, R₂, R₃, indépendamment les uns des autres représentent un atome d'hydrogène ; un atome d'halogène ; un groupement aryle en C₆-C₃₀ ; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino ; un groupement alkyl(C₁-C₆)hydroxyalkyl(C₁-C₆)amino , un groupement alcoxy(C₁-C₆); un groupement alcoxy(C₁-C₆)carbonyle ; un groupement carboxyalcoxy en C₁-C₆ ; un groupement pipéridinosulfonyle ; un groupement pyrrolidino ; un groupement alkyle(C₁-C₆)halogénoalkyle(C₁-C₆)amino ; un groupement benzoylalkyle(C₁-C₆) ; un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C₁-C₆, un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène, ce radical alkyle pouvant être interrompu par un hétéroatome ; les substituants R₁, R₂, R₃ peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄).

Le composé fluorescent peut être un dérivé stylbénique, par exemple de formule : 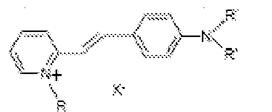 dans laquelle :
- R représente un radical méthyle ou éthyle ;
- R' représente un radical méthyle, et
- X⁻ représente un anion du type chlorure, iodure, sulfate, méthosulfate, acétate ou perchlorate.

A titre d'exemple de composé de ce type on peut citer le Photosensitiving Dye NK-557 commercialisé par la société UBICHEM, pour lequel R représente un radical éthyle, R' un radical méthyle et X⁻ un iodure.

Le composé fluorescent peut être un dérivé méthynique comme : 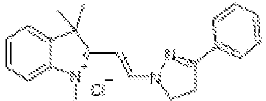 où un dérivé oxazine ou thiazine de formule générale :

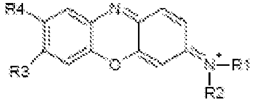

On peut citer aussi les dérivés dicyanopyrazines (de la société Nippon Paint), les dérivés de naphtolactames, les dérivés azalactones, les rhodamines, les dérivés de xanthènes.

On peut également utiliser des pigments ou particules minérales (MgO, TiO₂, ZnO, Ca(OH)₂...) ou organiques (latex...) comprenant en leur coeur ou sur leur surface de tels composés.

Le composé fluorescent peut aussi être un composé semi-conducteur qui, par exemple sous la forme de petites particules, appelées quantum dots, présente un effet fluorescent.

Les quantum dots sont des nanoparticules semi-conductrices luminescentes capables d'émettre, sous excitation lumineuse, un rayonnement présentant une longueur d'onde comprise entre 400 nm et 700 nm. Ces nanoparticules peuvent être fabriquées selon les procédés décrits par exemple dans les brevets US 6 225 198 ou US 5 990 479, dans les publications qui y sont citées, ainsi que dans les publications suivantes : Dabboussi B.O. et al. "(CdSe)ZnS core-shell quantum dots: synthesis and characterisation of a size series of highly luminescent nanocristallites" Journal of physical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

Des composés fluorescents préférés sont ceux émettant les couleurs bleues, vertes ou rouge.

De préférence, le ou les composés fluorescents utilisés en tant qu'agents optiques dans l'invention sont choisis parmi les stylbéniques.

### Actifs

La composition peut comporter au moins un ou plusieurs actifs additionnels. Le(ou les) actif(s) cosmétique(s) selon l'invention peuvent être choisi(s) parmi :
- les agents anti-âge/anti-rides,
- les agents hydratants,
- les agents anti-radicalaires,
- les agents agissant sur la microcirculation,
- les agents agissant sur le métabolisme énergétiques des cellules,
- de l'acide hyaluronique,
- les agents anti-glycation,
- les inhibiteurs de NO-synthase,
- les agents stimulant la synthèse de macromolécules dermiques ou épidermiques et/ou empêchant leur dégradation,
- les agents stimulant la prolifération des fibroblastes et/ou des kératinocytes,
- les agents myorelaxants,
- les saccharides,
- les oligosaccharides, les polysaccharides hydrolysés ou non, modifiés ou non,
- les acides aminés, les oligopeptides, les peptides, les protéines hydrolysées ou non, modifiées ou non, les polyacides aminés, les enzymes,
- les cires animales, végétales ou minérales,
- les céramides et les pseudo-céramides,
- les acides organiques hydroxylés,
- les antioxydants et les agents anti-radicaux libres,
- les agents apaisants,
- les polymères anioniques solubles ou dispersés,
- les polymères non-ioniques solubles ou dispersés,
- les particules, et
- leurs mélanges.

Parmi les composés additionnels anti-âge, on peut tout particulièrement mentionner les vitamines, telle que la vitamine A, la vitamine C, la biotine, les vitamines B, telles que l'acide folique, le panthénol, la niacinamide, les acides de fruits, les α-hydroxyacides, la déhydroépiandrostérone (DHEA), des micro-nutriments, la phosphatidylcholine, des agents tenseurs, comme le 2-(diméthylamino)éthanol, la procaïne ou le 2-(diéthylamino)éthyl-4-aminobenzoate).

Avantageusement, la composition injectable selon l'invention peut contenir en outre au moins un actif additionnel et notamment un actif anti-rides.

Des exemples d'actifs anti-rides utilisables selon l'invention sont l'acide ascorbique et ses dérivés, tels que l'ascorbyl phosphate de magnésium et le glucoside d'ascorbyle ; l'adénosine et ses dérivés notamment non phosphatés ; le tocophérol et ses dérivés, tels que l'acétate de tocophéryle ; l'acide nicotinique et ses précurseurs, tels que la nicotinamide ; l'ubiquinone ; le glutathion et ses précurseurs, tels que l'acide L-2-oxothiazolidine-4-carboxylique ; les composés C-glycoside et leurs dérivés, notamment ceux décrits dans la demande WO 02/051828 ; les extraits de plantes et notamment les extraits de criste marine et de feuille d'olivier, ainsi que les protéines végétales et leurs hydrolysats, tels que les hydrolysats de protéines de riz ou de soja ; les extraits d'algues et en particulier de laminaires ; les extraits bactériens ; les sapogénines, telles que la diosgénine et les extraits de Dioscorées, en particulier de Wild Yam, en contenant ; les α-hydroxyacides ; les β-hydroxyacides, tels que l'acide salicylique et l'acide n-octanoyl-5-salicylique ; les oligopeptides et pseudodipeptides et leurs dérivés acylés, en particulier l'acide {2-[acetyl-(3-trifluoromethyl-phenyl)-amino]-3-methyl-butyrylamino} acétique et les lipopeptides commercialisés par la société SEDERMA sous les dénominations commerciales Matrixyl 500 et Matrixyl 3000 ; le lycopène ; le rhamnose ; les sels de manganèse et de magnésium, en particulier les gluconates ; et leurs mélanges. On peut citer aussi les vitamines, telles que par exemple les vitamines B3 ou PP, B5, E, K1.

Selon un mode de réalisation particulier, ce ou ces composés additionnels sont choisis parmi les micronutriments, les vitamines, notamment les dérivés de vitamines B, tels que le panthénol, la niacinamide ou l'acide folique, la biotine et en particulier l'acide hyaluronique ou l'un de ses dérivés non réticulé ou faiblement réticulé.

La composition peut également comporter des actifs biologiques, comme le botox, le collagène, l'acide hyaluronique et les anti-inflammatoires.

La composition peut comporter des cellules souches.

La présence d'actif biologique dans la composition peut permettre de limiter la diffusion de la composition et de faciliter la résorption de la composition en excès.

La quantité d'actifs dépend bien évidemment de la nature de l'actif et de l'effet recherché, mais celui-ci représente généralement de 0,01 à 10 %, voire de 0,1 à 5 %, ou même de 0,001 à 30 % du poids total de la composition.

### Fibres

La composition injectable selon l'invention peut en outre contenir des fibres.

Ces fibres par enchevêtrement assurent une bonne cohésion au matériau de comblement.

Par « fibre », il faut comprendre un objet de longueur L et de diamètre D tel que L soit supérieur à D, et de préférence, très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Les fibres utilisables dans une composition de l'invention peuvent être des fibres d'origine synthétique ou naturelle, minérale ou organique, et elles peuvent être souples ou rigides.

Elles peuvent être courtes ou longues, unitaires ou organisées par exemple tressées.

Leur forme peut être quelconque et notamment de section circulaire ou polygonale (carrée, hexagonale ou octogonale) selon l'application spécifique envisagée.

De préférence, les fibres utilisables dans une composition selon l'invention sont choisies parmi les fibres naturelles ou dérivées, telles que de la rayonne.

La composition peut comporter entre 1 % et 40 % en poids de fibres par rapport à son poids total.

### Charges

Les charges distinctes des fibres précitées, susceptibles d'être présentes dans une composition selon l'invention, peuvent être de toute forme, plaquettaires, sphériques, hémisphériques ou oblongues, quelle que soit la forme cristallographique (par exemple feuillet, cubique, hexagonale, orthorhombique, etc....).

A titre illustratif de ces charges, peuvent être cités le talc, le mica, la silice, le kaolin, les poudres de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique, les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique. On peut également utiliser des particules, qui ont la forme de portions de sphères creuses, telles que décrites dans les demandes de brevet JP-2003 128 788 et JP-2000 191 789.

### Administration de la composition

La composition peut être injectée en utilisant un quelconque des modes connus de l'homme du métier.

Le système peut comporter un dispositif d'injection adapté à une injection intraépidermique et/ou intradermique et/ou sous-cutanée.

Dans un exemple particulier, l'injection peut s'effectuer sous l'ongle.

Le dispositif d'injection peut être choisi parmi une seringue, un ensemble de microseringues, un dispositif laser, hydraulique ou un scalpel.

Dans une variante de réalisation, le dispositif d'injection peut être adapté à la technique de la mésothérapie.

La mésothérapie est une technique de traitement par injection intraépidermique et/ou intradermique et/ou sous-cutanée de produit(s) actif(s).

La composition est administrée selon cette technique par injection sous forme de multiples gouttelettes de faible taille au niveau de l'épiderme, de la jonction dermo-épidermique et/ou du derme afin, notamment, de réaliser un nappage sous-cutané. La technique de mésothérapie est notamment décrite dans l'ouvrage « Traité de mésothérapie » de Jacques LE COZ, édition Masson, 2004.

La mésothérapie faite sur le visage est également appelée mésolift, ou également sous le terme anglosaxon de « mesoglow ».

De façon alternative, le système de traitement peut comporter un système de microperforation avec lequel on peut microtrouer la peau et/ou les lèvres, puis appliquer la composition par voie topique pour qu'elle diffuse naturellement la peau et/ou les lèvres.

L'administration par injection intraépidermique et/ou intradermique et/ou sous-cutanée selon l'invention vise à injecter une composition de l'invention dans une région épidermique, dermo-épidermique et/ou dermique.

Le système de traitement selon l'invention peut comporter tout moyen d'injection convenant à une injection intraépidermique et/ou intradermique et/ou sous-cutanée.

Ainsi, l'injection peut être effectuée à travers une aiguille habituellement utilisée pour réaliser une injection intraépidermique et/ou intradermique et/ou sous-cutanée, par exemple convenant pour la mésothérapie.

Une aiguille d'un système selon l'invention, peut présenter un diamètre variant de 0,26 à 0,4 mm, et une longueur variant de 4 à 13 mm.

En particulier, l'aiguille convenant à l'invention peut présenter un diamètre de 0,3 mm et une longueur de 6 mm.

L'aiguille est avantageusement à usage unique.

Avantageusement, l'aiguille est associée à une seringue ou tout autre dispositif permettant de délivrer à travers l'aiguille ladite composition injectable. Selon une variante de réalisation, un cathéter peut être intercalé entre l'aiguille et la seringue.

De façon connue, la seringue peut être actionnée manuellement par le praticien ou bien par un support de seringue comme les pistolets.

On peut utiliser un pistolet injecteur à compression hydropneumatique à hausse fréquence d'injection.

Ainsi, un pistolet injecteur convenant à l'invention peut être, par exemple, tel que décrit dans les brevets EP 1 208 858 ou US 5 300 029 introduits ici par référence.

Par exemple un pistolet injecteur convenant à l'invention peut être le MesoMega^{®}, l'Inderm PSI^{®}, le Pistor 4^{®}, le DHN3^{®} ou le Rofil Meso Gun U225^{®} commercialisé par la société Rofil (Biophymed).

En fonction du dispositif d'injection mis en oeuvre, la composition selon l'invention peut être injectée sous forme de gouttelettes.

Avantageusement, les gouttelettes peuvent posséder un volume moyen variant de 0,01 à 0,2 ml en particulier de 0,05 à 0,15 ml.

Le système de traitement peut comporter un pistolet injecteur, notamment un pistolet injecteur à compression hydropneumatique à haute fréquence d'injection.

Selon un autre aspect, l'invention a pour objet un ensemble de traitement des imperfections esthétiques de la peau et/ou des lèvres comportant :
i. un système de traitement tel que décrit précédemment,
ii. un système d'éclairage apte à faire photoréticuler le composé.

Selon encore un autre de ses aspects, l'invention a pour objet un ensemble de traitement des imperfections esthétiques de la peau et/ou des lèvres comportant en outre au moins :
i. un système d'analyse d'au moins une zone à traiter,
ii. une source d'autres moyens d'activation choisi parmi une source de chaleur, de microondes, d'ondes sonores, une source lumineuse ne facilitant pas la réticulation du ou des composés,
iii. un système d'application d'une contrainte avant ou pendant l'illumination,
iv. un dispositif d'élimination des composés n'ayant pas réagi.

### Illumination pour réticulation

La réticulation peut se faire à lumière ambiante ou bien de préférence avec une lumière artificielle, telle qu'un éclairage avec une lampe, un flash, un laser, des LEDs, par exemple sous forme d'une matrice de LEDs.

L'injection suivie de l'illumination permet de réaliser un matériau résistant dans la peau et/ou les lèvres, permettant ainsi de réduire les creux ou donner plus de volume.

Le système d'éclairage peut être agencé pour illuminer la zone de traitement de façon externe, à travers la peau et/ou les lèvres. Dans une variante, la lumière peut être amenée dans ou sous la peau et/ou les lèvres.

L'ensemble de traitement peut comporter une fibre optique pour éclairer la zone à traiter de façon interne.

L'ensemble de traitement peut comporter une source lumineuse avec un rayonnement dans le domaine visible et/ou un rayonnement dans l'UV et de préférence un rayonnement dans le visible (lumière blanche).

La lumière émise peut être ou non monochromatique. La longueur d'onde de la lumière émise est de préférence centrée sur 365 nm, en particulier comprise entre 400 nm et 700 nm, et mieux entre 365 nm et 550 nm.

La réticulation peut s'initier par l'illumination sans nécessiter de photoinitiateur.

La réticulation peut se produire avec une intensité lumineuse réduite, le système d'éclairage peut produire par exemple une intensité lumineuse inférieure à 50 mJ/cm² ou même à 10 J/cm².

De préférence, le système d'éclairage a une intensité lumineuse comprise entre 1 mW et 10 W, les temps d'exposition étant adaptés en conséquence.

En particulier, l'énergie lumineuse dans l'UV est de préférence inférieure à 10J/cm².

La double caractéristique de l'absence de photoinitiateur et d'intensité lumineuse relativement faible est particulièrement avantageuse car elle permet de limiter les effets néfastes d'initiateurs agressifs ou d'exposition prolongée à une lumière intense, en particulier dans les longueurs d'onde UV.

L'illumination peut être globale ou localisée, voire être structurée et représenter une image.

La structuration peut être réalisée de façon à traiter certaines zones particulières, comme le fond des rides, le bord des lèvres, les creux (cicatrices d'acné, pores, vergetures...).

Plusieurs approches de structuration sont possibles sur le plan technique : illuminer la surface avec une « image » projetée ou illuminer la surface avec un rayon mobile, par exemple un rayon laser orienté par un jeu de miroirs mobiles, le mouvement du rayon reproduisant une image.

La première approche implique un imageur : une source lumineuse, un support portant une image, par exemple un négatif, et un système de focalisation.

### Imageur

Le système de radiation peut comporter un imageur ou un rayon mobile reproduisant une image. Le système d'éclairage peut comporter un imageur électronique matriciel adressable tel que par exemple un imageur décrit dans le document n° FR 2 942 405.

Un imageur matriciel adressable permet de projeter une image pixellisée dont la résolution est par exemple supérieure à 10 par 10 pixels et préférentiellement supérieur à 10 par 100 pixels.

Lorsque l'imageur est un imageur électronique matriciel adressable, l'image formée sur la zone à traiter est formée de pixels qui sont allumés ou éteints, éventuellement chacun selon un niveau de gris prédéfini.

Un calculateur peut déterminer l'image numérique sur la base de laquelle l'imageur électronique est commandé, en particulier le niveau de gris de chaque pixel, ainsi éventuellement que la longueur d'onde dominante de la lumière au niveau de chaque pixel.

L'imageur matriciel adressable peut être réalisé sur la base de plusieurs technologies.

On peut employer la technologie dite DLP (*Digital Light Proccessing*) inventée par la société TEXAS INSTRUMENTS, qui utilise une puce DMD (*Digital Micromiror Device*) composée de milliers de micromiroirs qui peuvent être commandés individuellement en orientation sous l'effet d'une impulsion électrique et peuvent réfléchir ou non suivant leur orientation un faisceau lumineux incident afin de le renvoyer ou non vers la sortie optique de l'imageur. L'image à projeter est formée sur la matrice de miroirs.

Les niveaux de gris de chaque pixel (par exemple au nombre de 256 niveaux) peuvent être commandés en jouant sur le rapport cyclique.

L'irradiateur peut encore utiliser la technologie dite LCD.

L'irradiateur peut comporter au moins un miroir dichroïque.

Le système de projection peut encore être fondé sur la technologie dite LCOS à cristaux liquides sur silicium. La technologie LCD est dite transmissive puisque la lumière traverse un écran LCD alors que la technologie DLP est dite réflective, car la lumière est reflétée par les micromiroirs de la puce DMD. Dans la technologie LCOS, les miroirs des puces DMD sont remplacés par une surface réfléchissante recouverte d'une couche de cristaux liquides, qui peuvent être commutés entre un état passant et un état bloquant. En modulant la fréquence d'ouverture et de fermeture des cristaux liquides, on peut faire varier le niveau de gris d'un pixel.

D'une manière générale, l'image délivrée par l'imageur matriciel adressable comporte une matrice de pixels dont les niveaux de gris sont individuellement adressables, chaque niveau de gris étant par exemple codé sur au moins 4 bits, mieux 8 bits. La lumière associée à chaque pixel peut également faire l'objet d'un codage, le cas échéant.

L'image à projeter peut être fournie à l'imageur électronique sous forme de signal vidéo VGA, SVGA, composite, HDMI, SVIDEO, YC_{B}C_{R}, optique, entre autres standards, ou sous forme de fichier informatique image ou vidéo, par exemple .jpeg, .pdf, .ppt, ... Sur ces images, lorsqu'elles ne sont pas monochromes, une couleur prédéfinie sur l'image dans le fichier peut commander le niveau d'UV ou de proche UV, par exemple.

L'imageur électronique est avantageusement réalisé de façon à pouvoir changer la nature de la lumière émise sans changer l'image ; par exemple, les pixels de l'image conservent leurs niveaux de gris et seul le spectre d'émission de la source utilisée en amont change. Cela peut permettre de visualiser une image sur la zone à traiter et ensuite de la révéler, simplement en modifiant le spectre d'émission de la source.

### Analyse de la zone à traiter et choix de l'image projetée

Le système de traitement peut comporter un système d'analyse des zones à traiter. Par exemple, le système d'analyse scanne la peau et/ou les lèvres et détermine par intervention humaine ou automatique les zones à irradier.

Le système de traitement peut être configuré pour détecter de façon automatique un défaut cutané sur la zone à traiter et l'imageur peut être commandé en fonction de la nature du défaut détecté.

Le système de traitement peut être doté de fonctions de reconnaissance particulières, destinées par exemple à reconnaître les défauts, par exemple : rides, crevasses, vergetures, veines, reliefs en creux ou en bosse, tels que des cicatrices, asymétries.

Les défauts peuvent être détectés par analyse d'image et/ou du relief. L'analyse d'image peut être une analyse d'image 3D. Le système de traitement peut comporter des moyens d'acquisition de la forme 3D du visage ou de la partie du corps à traiter.

Le choix peut par exemple se faire suivant une logique de rétablissement de la symétrie pour les visages présentant des asymétries et/ou selon une logique d'ombres et de lumières, par laquelle on peut rendre plus rond un visage trop anguleux ou l'inverse, ou rectifier des proportions peu esthétiques ou naturelles.

Le système de traitement peut être configuré pour faire défiler plusieurs modèles picturaux, sous forme de simulations, afin de permettre à une personne de choisir parmi celles-ci le modèle à réaliser.

Le système de traitement peut offrir la possibilité d'essayer rapidement toutes sortes de modèles, par projection directement sur le visage. Ainsi, la personne peut se rendre compte, en version réelle, de l'adéquation de ce ou ces modèles sur elle.

Le système de traitement peut être configuré pour prendre une image, par exemple à l'aide du dispositif d'acquisition optique précité, en extraire éventuellement une partie correspondant à la zone à traiter, et permettre de rectifier le cas échéant cette image pour en améliorer le rendu une fois projetée.

Le système de traitement utilisé est préférentiellement configuré pour permettre à l'utilisateur, à partir d'une image projetée sur le visage ou sur toute autre zone à traiter, d'en rectifier la forme, par exemple par agrandissement, rétrécissement, dans une ou deux dimensions. Les modifications peuvent aussi être plus complexes. Ainsi, on peut par exemple rectifier une partie de l'image, étirer une zone particulière, changer la taille des traits. En cela, on peut utiliser les outils habituellement présents dans les logiciels de réalisation et de retouche d'image, tels que Photoshop^{®} par exemple. La retouche de l'image peut s'effectuer, le cas échéant, grâce à un renvoi d'information par le dispositif d'acquisition optique, le calculateur pouvant connaître le rendu de l'image projetée et la modifier automatiquement jusqu'à atteindre le résultat souhaité, lors de l'exécution d'une boucle logicielle par le système de traitement.

### Autre moyen d'activation

L'ensemble de traitement peut comporter au moins une source d'un autre moyen d'activation choisi parmi une source de chaleur, de microondes, d'ondes sonores, une source lumineuse ne facilitant pas la réticulation du ou des composé(s).

### Dispositif de modelage

L'ensemble de traitement des défauts/imperfections esthétiques de la peau et/ou des lèvres peut comporter un système d'application d'une contrainte avant ou pendant l'illumination.

Un système/dispositif tout intégré peut être utilisé.

### Procédé de traitement

Dans un autre de ses aspects, l'invention a pour objet un procédé cosmétique de traitement de la peau et/ou des lèvres comprenant les étapes suivantes :
i. administration dans ou à travers la peau et/ou les lèvres d'une composition telle que définie précédemment,
ii. illumination de la peau et/ou des lèvres traitées selon l'étape i. pour réticuler la composition.

Comme précisé ci-dessus, on peut également utiliser un traceur (groupe fluorescent) pour aider le praticien à localiser la composition une fois injectée. Ce faisant, cela l'aide à irradier toute la zone où est la composition.

De préférence, le traceur change d'aspect (changement de couleur ou devient incolore) lorsque la polymérisation a eu lieu.

Dans un mode particulier, le procédé comporte après l'étape d'injection de la composition une étape de modelage, par exemple par aspiration, lissage, extension, traction, ou compression. Ceci est un grand avantage, car il permet des effets de modelage.

L'étape de modelage peut être antérieure et/ou simultanée avec l'illumination.

Durant cette étape, la zone de traitement peut être, au moins en partie, soumise à une autre action énergétique telle que, application de chaleur, microondes, laser, illumination à une longueur d'onde ne provoquant pas de réticulation d'un des composés de la composition.

Selon un mode de réalisation, l'étape d'injection peut être réitérée sur tout ou partie de la surface à traiter. La répétition des injections permet de créer un nappage de sorte que la composition de l'invention est distribuée de manière homogène dans la région cutanée à traiter.

Une composition de l'invention peut être avantageusement injectée au niveau des rides et des ridules.

Il y a plusieurs techniques d'injection. Par exemple, dans le cas d'une injection par mésothérapie, on peut utiliser la micro-poncture (piqures à distance rapprochée) ou le multipoint (piqures en plusieurs points plus espacés).

Les injections répétées peuvent être également réalisées au point par point manuellement. Les points de piqures peuvent être espacés par exemple d'une distance variant de 5 mm à 1 cm.

Selon un mode de réalisation, un procédé de l'invention peut être effectué en plusieurs séances espacées l'une de l'autre de quelques jours par exemple de 1, 2, 3, 4, 5 ou 6 jours à quelques semaines, par exemple 1, 2, 3, 4 semaines.

Selon un mode de réalisation, la ou les étape(s) d'administration par injection peuvent être avantageusement effectuée(s) au moyen d'un pistolet injecteur, notamment un pistolet injecteur à compression hydropneumatique à haute fréquence d'injection tel que défini précédemment.

Le procédé peut comporter une étape d'élimination de la partie de la composition n'ayant pas réticulé. Par exemple, on peut percer la peau et aspirer le composé resté fluide.

On peut après illumination, appliquer, par voie topique ou injectable, un tiers composé destiné à réagir avec les fonctions n'ayant pas réagi, pour éviter une photoréticulation ultérieure. Par exemple, on injecte un produit monomère de la même fonction.

Selon un mode particulier de réalisation, les régions cutanées avantageusement traitées par un procédé de l'invention peuvent être la peau du visage et du cou, du décolleté, de l'abdomen, des jambes.

Le procédé peut comporter une première illumination d'une zone de traitement correspondant à une partie seulement de la peau et/ou des lèvres ayant reçu la composition, partie que l'on veut traiter différemment, notamment plus fortement, et une seconde illumination sur l'ensemble de la peau et/ou des lèvres ayant reçu la composition.

Selon l'invention, le procédé de traitement des défauts/imperfections esthétiques de la peau et/ou des lèvres peut comporter au moins une étape visant à :
- modeler la peau, les lèvres,
- combler des rides ou sillons de la peau,
- réparer la peau, par exemple les vergetures, les cicatrices notamment d'acné,
- retendre la peau,
- rehausser des parties du corps affaissées,
- augmenter le volume de certaines parties du corps en particulier du visage, notamment le bord des lèvres,
- renforcer le maintien d'implants, de fillers, ou de composés actifs.

La présente invention peut être mise en oeuvre pour le traitement, la prévention et/ou la réduction des effets inesthétiques du vieillissement de la peau et/ou des lèvres. On entend, au sens de l'invention, par « prévention », le fait de diminuer le risque de survenue d'un phénomène. On peut par exemple créer une couche sous-cutanée destinée à prévenir le relâchement de la peau.

Le procédé conforme à la présente invention s'avère ainsi avantageux pour le traitement du vieillissement cutané et/ou le photo-vieillissement cutané.

Il permet aussi de prévenir et/ou traiter les signes cutanés du vieillissement ou du photo-vieillissement choisis parmi la peau ridée, la peau présentant une altération de ses propriétés viscoélastiques ou biomécaniques, la peau présentant une altération dans la cohésion de ses tissus, la peau amincie, la peau présentant une altération de son aspect de surface et/ou de grain, dans un dispositif pour administration sous-cutanée intradermique et/ou intraépidermique.

La présente invention peut être par exemple utilisée en prévention du relâchement de la peau.

La peau et/ou les lèvres peuvent être prétraitées par une composition différente, non couverte par l'invention, avant administration de la composition comportant le groupe destiné à photoréticuler.

La peau et/ou les lèvres peuvent être traitées par une composition différente, non couverte par l'invention, après administration de la composition comportant le groupe destiné à photoréticuler et avant illumination.

La peau et/ou les lèvres peuvent être traitées par une composition différente, non couverte par l'invention, après illumination et avant rinçage.

Après illumination et après rinçage, la peau et/ou les lèvres peuvent être dans une autre variante traitées par une composition différente, non couverte par l'invention.

Par exemple, on peut traiter la peau et/ou les lèvres avec des agents, réactifs ou non, améliorant l'adhésion du matériau ou des agents protecteurs notamment contre la lumière UV ou bien retardant l'usure du matériau.

L'invention pourra être mieux comprise à la lecture de la description détaillée qui va suivre, d'exemples de mise en oeuvre non limitatifs de l'invention et à l'examen du dessin annexé, sur lequel :
- les figures 1A et 1B représentent schématiquement deux méthodes d'utilisation d'un système de traitement selon l'invention,
- les figures 2A à 2C représentent des dispositifs d'injection d'un système de traitement selon l'invention,
- la figure 3 représente de manière schématique un ensemble de traitement, et
- les figures 4a et 4b représentent un système de modelage.

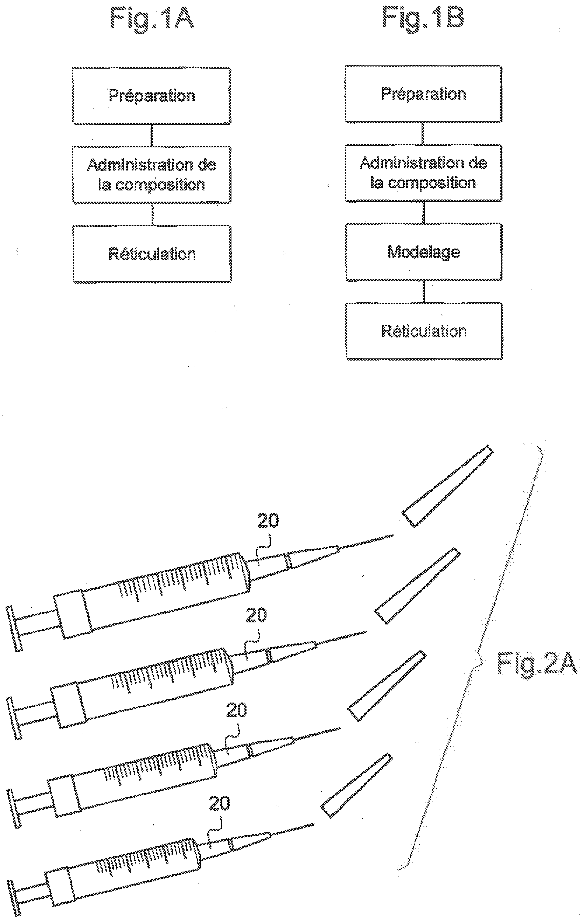

L'étape de préparation illustrée aux figures 1A et 1B correspond à préparer d'une part le matériel pour l'injection, dépendant de la technique choisie, et d'autre part la composition à injecter.

La composition peut être préparée *in situ* ou être pré-conditionnée, notamment dans un conditionnement stérile.

Le conditionnement est approprié au stockage, d'au moins une dose de la composition, le cas échéant, au mélange de celle-ci avec un milieu physiologiquement acceptable liquide, dans un environnement stérile.

Le conditionnement peut être agencé pour permettre par ailleurs le prélèvement de ladite composition à l'état de solution ou de dispersion.

Ainsi, selon un premier mode de réalisation, ledit conditionnement est sécable pour permettre le prélèvement de ladite composition.

Par exemple, il peut se présenter sous la forme d'une ampoule ou d'une capsule monodose, disposant d'une extrémité sécable.

Selon un deuxième mode de réalisation, ledit conditionnement dispose d'un opercule permettant une fermeture hermétique lors du stockage et transperçable par une aiguille au moment de l'utilisation.

Selon une variante de réalisation, ladite composition est formulée sous la forme d'une dispersion ou d'une solution au sein du conditionnement.

Selon une autre variante, ladite composition est présente à l'état de poudre.

Dans une telle variante, ledit conditionnement est propice à l'introduction d'un milieu physiologiquement acceptable fluide et stérile pour obtenir une solution et/ou dispersion de ladite composition dans des conditions stériles.

Un tel conditionnement peut également être bicompartimenté, l'un des compartiments étant dédié au stockage de ladite composition à l'état de poudre et l'autre à celui du milieu physiologiquement acceptable liquide devant lui être associé. Ces deux compartiments sont avantageusement séparés par un moyen de bouchage configuré pour être déplacé et permettre alors le mélange du contenu des deux compartiments.

Un tel dispositif est avantageusement dédié à un usage unique.

La figure 2A représente plusieurs seringues 20, pré remplies d'une dose de composition. Les doses correspondent par exemple respectivement à 1 ml, 1ml, 1ml et à 1ml de composition.

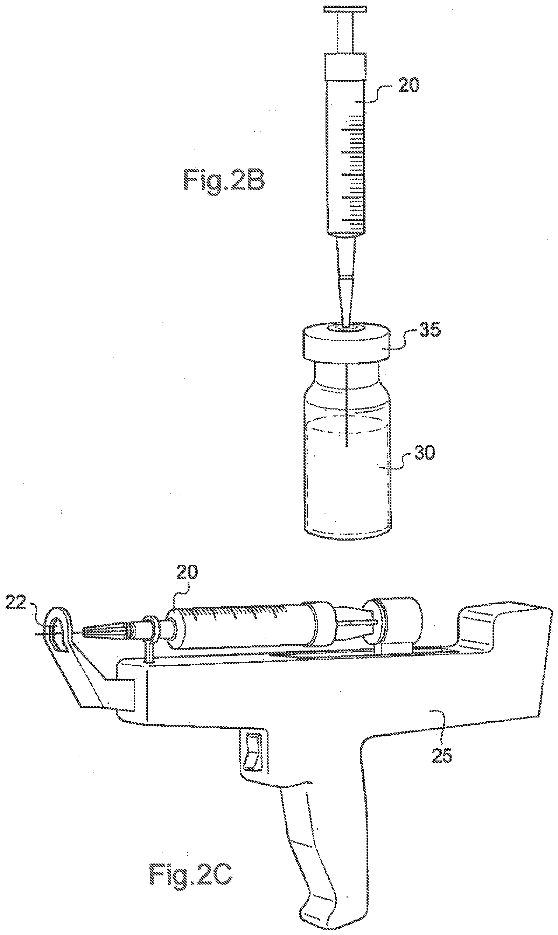

Dans la variante de la figure 2B, la seringue 20 n'est pas pré remplie et la composition est conditionnée dans un flacon stérile 30. Dans l'exemple illustré, le flacon est fermé hermétiquement par un organe de fermeture 35 réalisé en matière souple, apte à être percée par l'aiguille de la seringue 20. D'autres modes de fermeture sont envisageables.

La figure 2C représente en perspective un pistolet 25 utilisé pour une administration par mésothérapie de la composition. La seringue 20 et la mire 22 sont de préférence à usage unique.

Les exemples représentés ne sont pas limitatifs. L'homme du métier peut choisir toute technique d'injection ou d'application intradermique connue.

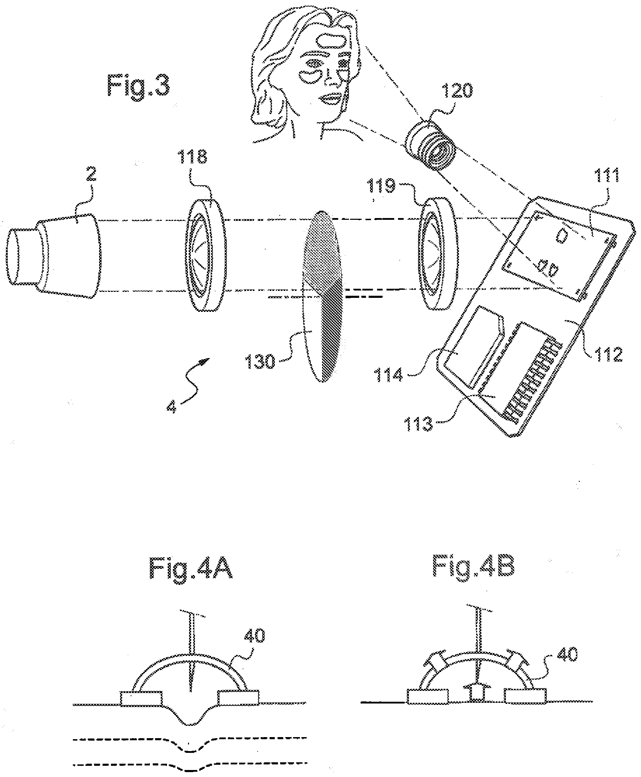

Après administration de la composition, la zone à traiter, par exemple le visage comme illustré à la figure 3, est éclairée pour faire réticuler le composé.

Dans l'exemple illustré, la réticulation est produite par une source artificielle de lumière, dans une autre variante, la réticulation peut se faire à la lumière du jour.

Le système de traitement de la figure 3 comporte de façon optionnelle un imageur électronique 4 réalisé selon la technologie DLP, utilisant une puce DMD référencée 111. Cette dernière peut être fixée sur une platine 112 qui peut comporter par ailleurs un processeur 113 pour commander la puce, ainsi qu'éventuellement une mémoire 114. Dans l'exemple illustré, la puce est représentée sur la même platine que le processeur 113 et la mémoire 114, mais ces derniers peuvent être disposés autrement.

L'imageur 4 représenté à la figure 3 reçoit de la lumière d'une source 2 qui peut être une source pouvant émettre à la fois dans l'UV et/ou dans le visible ou une source pouvant émettre sélectivement dans le visible ou dans l'UV.

La source 2 peut être une lampe halogène à émission dans les spectres UV et visible, une lampe à décharge ou une ou plusieurs LEDs capables d'émettre par exemple dans l'UV et en lumière blanche ou dans une couleur donnée.

L'imageur 4 peut comporter, comme illustré, des optiques 118, 119 et 120 respectivement pour condenser la lumière, la focaliser sur la puce DMD et assurer la mise au point sur la zone à traiter.

Lorsque la source 2 présente un spectre d'émission à la fois dans l'UV et dans le visible, l'imageur 4 peut comporter, comme illustré, une roue à filtres 130, qui intercepte le faisceau lumineux par exemple entre l'optique de condensation 118 et celle de focalisation 119. La puce reçoit selon la position de la roue à filtres 130 une lumière UV ou une lumière visible, qui est ensuite dirigée vers la sortie optique. On peut ainsi former sur la zone à traiter une image sélectivement en lumière visible et/ou dans l'UV.

Dans une variante non représentée, l'irradiateur utilise plusieurs puces DMD fixées sur un prisme.

On peut utiliser l'agencement illustré à la figure 3 en remplaçant les puces DMD par des puces LCOS.

Une fois la composition injectée, la méthode illustrée à la figure 1B comporte en outre une étape de modelage destinée à forcer le matériau à donner une meilleure conformation à la peau.

Cette étape peut être effectuée par plusieurs moyens, éventuellement combinés, notamment par aspiration, lissage, extension, actions énergétiques telles que radiation par microondes ou laser.

Le modelage peut s'effectuer à faible lumière, voire dans le noir, avant le début de la phase de réticulation.

Dans une autre variante, le modelage peut s'effectuer simultanément à la réticulation, au moins en partie, par exemple commencer préalablement et continuer pendant l'étape de réticulation.

Dans une variante, le modelage est réalisé par massage de la zone de traitement afin de préformer la composition avant réticulation. Le massage peut se faire manuellement et/ou à l'aide d'un organe de massage.

Les figures 4A et 4B illustrent un système d'application d'une contrainte 40, posé directement sur la peau au niveau d'une ride ou d'un sillon comme illustré figure 4A. Le système génère des forces qui ont pour effet de retendre la peau.

Le procédé illustré figure 1B comporte en outre de façon optionnelle une étape d'élimination de la partie de la composition n'ayant pas réticulé.

D'autres caractéristiques et avantages de l'invention ressortiront des exemples qui suivent donnés à titre illustratif et non limitatif.

Dans ce qui suit, les proportions sont données en pourcentage pondéral, sauf indication contraire.

L'expression « comportant un » est synonyme de « comportant au moins un » et « compris entre » doit s'entendre bornes incluses.

Les exemples suivants sont donnés à titre illustratif et non limitatif de la présente invention.

L'invention n'est pas limitée aux dispositifs illustrés.

### Exemple 1 : Synthèse de gomme de xanthane greffée de groupements stylbazolium

De la gomme de xanthane desacétylée est préparée en dissolvant 0,2 % en poids de gomme de xanthane (RHODICARE^{®} XC de chez Rhodia) dans de l'eau et en ajoutant 0,025 Mole de potasse et 0,1 % en poids de chlorure de potassium pendant 2h30 à température ambiante, sous atmosphère d'azote.

La solution alcaline est ensuite neutralisée avec 0,05 Mole d'acide chlorhydrique afin d'ajuster le pH à 6,5.

La solution est ensuite dialysée avec de l'eau distillée en utilisant un tube de dialyse (Snakeskin TM Pleated Dialysis Tubing, Pierce, Rockford, I11., U.S.A.), et la xanthane desacétylée est récupérée par lyophilisation.

La gomme de xanthane desacétylée est redissoute dans 20 ml d'eau.

0,2 g d'acide phosphorique concentré et 0,1 g de composé chlorure (1) de formule : 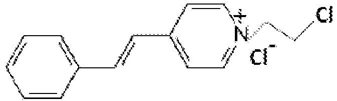 sont dissous dans 1 ml d'eau et y sont ajoutés.

Le mélange est maintenu sous agitation à température ambiante pendant une nuit.

Le mélange est ensuite précipité avec de l'acétone et lavé au méthanol contenant de l'ammoniaque.

### Synthèse du composé chlorure (1) de formule :

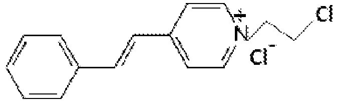

Un mélange de 4-styrylpyridine (2.3 g, 12.5 mmol) (J. L. R. Williams, R. E. Adel, J. M. Carlson, G. A. Reynolds, D. G. Borden, J. A. Ford, J. Org. Chem., 1963, 28, 387) et de 1,2-dichloroéthane (5 g, 50 mmol) a été chauffé à l'abri de la lumière pendant 4 heures à 150 °C à l'aide d'un bain d'huile. Le mélange est ensuite dissous en le chauffant dans 30 mL d'éthanol anhydre, puis refroidi à 5 °C.

Le précipité obtenu a été filtré, lavé avec du chloroforme et séché à l'air.

### Exemple 2 : Synthèse d'amidon greffé de groupements stylbazolium

20 g de sulfate de sodium et 1,5 g de soude sont dissous dans 75 ml d'eau et chauffés à 40 °C.

50 g d'amidon de maïs (Farma® CS 3757 commercialisé par Corn products international) sont ajoutés rapidement sous agitation.

4 g de composé chlorure (1) est ajouté en maintenant le pH au dessus de 11,6. Le mélange est agité pendant 24 heures à 40 °C. La réaction est ensuite refroidie à la température ambiante et le pH ajusté à 7 avec de l'acide chlorhydrique.

L'amidon modifié est ensuite filtré et le filtrat lavé avec de l'eau et séché à l'air.

### Exemple 3 : Synthèse d'alginate greffé de groupements stylbazolium

1 g d'alginate de sodium (KELCOSOL NF commercialisé par FMC corporation) est dissous dans 100 ml d'eau. Le pH de la solution est ensuite ajusté à 11,6 avec de la soude.

0,1 g de composé chlorure (1) dissous dans 1 ml d'eau est ajouté au milieu réactionnel en maintenant le pH à 11,6. Le mélange est agité pendant 24 heures à 40 °C puis refroidi à la température ambiante et le pH ajusté à 7 avec de l'acide chlorhydrique.

Le mélange est précipité avec de l'acétone, et le précipité est lavé avec du méthanol puis séché à l'air.

### Exemple 4 : Synthèse de chitosane greffé de groupements stylbazolium

Le chitosane greffé est obtenu de la même façon que l'alginate décrit précédemment en utilisant du chitosane (CHITOCLEAR SC342 de chez Primex).

### Exemple 5

On prépare la composition suivante qui est ensuite conditionnée dans un flacon sous atmosphère stérile.

| **Composés** | **Quantité (% pondéral)** |
|---|---|
| Alcool polyvinylique avec groupes pendants N-méthyl styryl pyridium sous forme de sel de méthylsulfate commercialisé par la société Polymer Science sous la dénomination PS22570 | 6,9 |
| Eau distillée | 83,1 |
| Rhamnose | 5 |
| Glycerol | 5 |

Cette composition injectable est utilisée pour un traitement de l'épiderme ridé par la technique d'injection avec une seringue à une profondeur d'0,5 mm sous la zone surface.

La zone à traiter est ensuite éclairée par un imageur avec une première longueur d'onde dominante dans le visible de 8,5 mW/cm² pendant 120 s, soit environ 1J/cm².

L'ensemble de la zone ayant reçu la composition est ensuite éclairée par un imageur avec une seconde longueur d'onde dominante dans l'UVA de 12 mW/cm² pendant 120 s.

L'excès de composition est éliminé par tamponnage. On observe après photopolymérisation du polymère injecté une diminution des rides à la surface de la peau.

Un autre mode de réalisation est décrit ci-après.

La composition injectable est utilisée pour un traitement de l'épiderme par la technique d'injection avec une seringue à une profondeur d'0,3 mm sous la zone surface d'une ride et l'injection est effectuée petit à petit en retirant la seringue.

La zone à traiter (0,2 cm sur 2 cm) est ensuite éclairée par un imageur avec un pointeur Led Laser 405 nm de 15mW (société Global Laser) défocalisée de façon à couvrir une surface de 0,4 cm x 0,4 cm (soit 0,16 cm²).

Un dispositif maintient à distance l'imageur de la peau.

Un balayage est réalisé (0,1 cm/s) pour traiter la zone sur les 2 cm de la longueur de la ride.

Ainsi, une surface de 0,4 cm sur 2 cm, soit 0,8 cm², est traitée.

Le traitement dure pendant 200 s, soit 4 J/cm².

### Exemple 6

On prépare la composition suivante qui est ensuite conditionnée dans un flacon sous atmosphère stérile :

| **Composés** | **Quantité (%)** |
|---|---|
| Gomme de xanthane greffée de groupements stylbazolium (Exemple 1) | 5 |
| Eau distillée | 90 |
| Glycérol | 5 |

La composition ainsi obtenue est utilisée pour un traitement de l'épiderme ridé conforme à celui décrit dans l'exemple 5.

### Exemple 7

On prépare la composition suivante qui est ensuite conditionnée dans un flacon sous atmosphère stérile :

| **Composés** | **Quantité (%)** |
|---|---|
| Amidon greffé de groupements stylbazolium (Exemple 2) | 5 |
| Eau distillée | 90 |
| Glycérol | 5 |

La composition ainsi obtenue est utilisée pour un traitement de l'épiderme ridé conforme à celui décrit dans l'exemple 5.

### Exemple 8

On prépare la composition suivante qui est ensuite conditionnée dans un flacon sous atmosphère stérile :

| **Composés** | **Quantité (%)** |
|---|---|
| Alginate greffé de groupements stylbazolium (Exemple 3) | 5 |
| Eau distillée | 90 |
| Glycérol | 5 |

La composition ainsi obtenue est utilisée pour un traitement de l'épiderme ridé conforme à celui décrit dans l'exemple 5.

### Exemple 9

On prépare la composition suivante qui est ensuite conditionnée dans un flacon sous atmosphère stérile :

| **Composés** | **Quantité (%)** |
|---|---|
| Chitosane greffé de groupements stylbazolium (Exemple 4) | 5 |
| Eau distillée | 90 |
| Glycérol | 5 |

La composition ainsi obtenue est utilisée pour un traitement de l'épiderme ridé conforme à celui décrit dans l'exemple 5.

## Revendications

1. Composition liquide et stérile, dédiée à une administration dans ou à travers la peau et/ou les lèvres, ladite composition comprenant dans un milieu physiologiquement acceptable au moins un composé photoréticulable, ledit composé comportant au moins un groupe photodimérisable activé possédant au moins une double liaison activée et choisi parmi :
a) les groupes photodimérisables portant une fonction stylbazolium de formule (Ia) ou (Ib) : 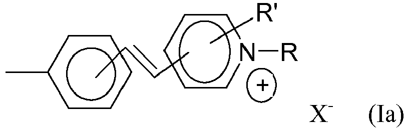 où
- R représente l'atome d'hydrogène, un groupement alkyle en C₁-C₄ ou hydroxyalkyle en C₁-C₄.
- R' représente l'atome d'hydrogène ou un groupement alkyle en C₁-C₄, et
- X⁻ désigne un ion choisi parmi les ions chlorures, bromures, iodures, perchlorates, tetrafluoroborates, méthylsulfate, phosphates, sulfates, méthanesulfonates, p-toluènesulfonate. 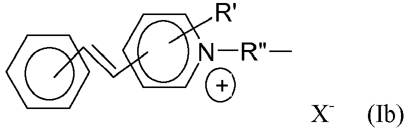
où
- R" désigne un radical alkylène divalent ayant de 2 à 8 atomes de carbone,
- R' représente l'atome d'hydrogène ou un groupement alkyle en C₁-C₄, et
- X⁻ ayant la même signification que celle décrite pour la formule (Ia) précédente.
b) les groupes photodimérisables portant une fonction styrylazolium de formule (II) : 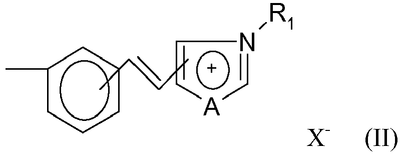 dans laquelle :
- R₁ désigne l'atome d'hydrogène, un groupement alkyle en C₁-C₄ ou hydroxyalkyle en C₁-C₄,
- A désigne un atome de soufre, un atome d'oxygène, ou un groupement NR' ou C(R')₂, R', R' représentant l'atome d'hydrogène ou un groupement alkyle en C₁-C₄, et
- X⁻ ayant la même signification que celle décrite pour la formule (Ia) précédente,
le ou les groupe(s) photodimérisable(s) étant portés par un polymère de type polyvinylacétate partiellement ou totalement hydrolysé, un polysaccharide ou un alcool polyvinylique.

2. Composition selon la revendication 1, **caractérisée en ce que** le groupe photodimérisable est un groupe photodimérisable portant une fonction stylbazolium de formule (Ia) : 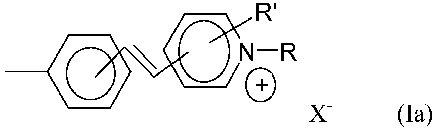 où
- R représente l'atome d'hydrogène, un groupement alkyle en C₁-C₄ ou hydroxyalkyle en C₁-C₄.
- R' représente l'atome d'hydrogène ou un groupement alkyle en C₁-C₄, et
- X⁻ désigne un ion choisi parmi les ions chlorures, bromures, iodures, perchlorates, tetrafluoroborates, méthylsulfate, phosphates, sulfates, méthanesulfonates, p-toluènesulfonate.

3. Composition selon l'une quelconque des revendications précédentes, **caractérisée en ce qu'**elle est dénuée de photoinitiateur et/ou d'initiateur chimique.

4. Composition selon l'une quelconque des revendications précédentes, dans laquelle les groupes photodimérisables sont portés par un alcool polyvinylique.

5. Composition selon l'une quelconque des revendications précédentes, dans laquelle le composé photoréticulable est un alcool polyvinylique fonctionnalisé en partie par une ou plusieurs fonction(s) hydroxyle(s) et une ou plusieurs fonction(s) de formule (III) :

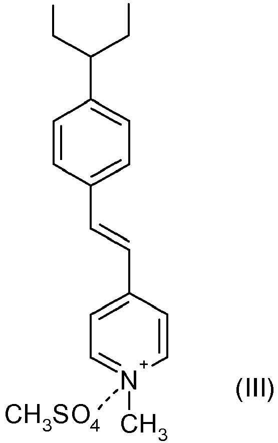

6. Composition selon l'une quelconque des revendications précédentes, **caractérisée en ce que** le degré de fonctionnalisation en motif photodimérisable est au moins de 0,1 %, voire au moins de 0,5 %, voire au moins de 2 %.

7. Composition selon l'une quelconque des revendications précédentes, dans laquelle le composé photoréticulable représente au moins 2 %, voire au moins 5 %, voire 10 % du volume total de la composition.

8. Composition selon l'une quelconque des revendications précédentes, comprenant en outre au moins un traceur optique ou un composé fluorescent.

9. Composition selon l'une quelconque des revendications précédentes, comprenant en outre au moins un actif additionnel, en particulier un actif anti-rides.

10. Procédé cosmétique de traitement de la peau et/ou des lèvres comprenant les étapes suivantes :
i. administration dans ou à travers la peau et/ou les lèvres d'une composition selon l'une quelconque des revendications 1 à 9, et
ii. illumination de la peau et/ou des lèvres traitées selon l'étape i. pour réticuler la composition.

11. Procédé cosmétique de traitement de la peau et/ou des lèvres selon la revendication 10, dans lequel l'illumination est une source lumineuse avec un rayonnement dans le domaine visible et/ou un rayonnement dans l'UV et de préférence un rayonnement dans le visible.

12. Système de traitement de la peau et/ou des lèvres comportant :
- un conditionnement contenant au moins une dose d'une composition selon l'une quelconque des revendications 1 à 9 et,
- un dispositif d'injection dans ou à travers la peau et/ou les lèvres ou un dispositif de microperforation de la peau et/ou des lèvres dédié à l'administration de ladite dose.

13. Système de traitement selon la revendication 12, comportant en outre un imageur.

14. Système de traitement selon l'une quelconque des revendications 12 et 13, comportant en outre un dispositif d'injection adapté à une injection intraépidermique et/ou intradermique et/ou sous-cutanée.

15. Système de traitement selon l'une quelconque des revendications 12 à 14, dans lequel le dispositif d'injection est choisi parmi une seringue, un ensemble de microseringues, un dispositif laser, hydraulique ou un scalpel.

16. Système de traitement selon l'une quelconque des revendications 12 à 14, dans lequel le dispositif d'injection est adapté à la technique de la mésothérapie.

17. Ensemble de traitement des imperfections esthétiques de la peau et/ou des lèvres comportant :
i. un système de traitement selon l'une quelconque des revendications 12 à 16, et
ii. un système d'éclairage apte à faire photoréticuler le composé.

18. Ensemble de traitement selon la revendication 17 comportant en outre au moins :
i. un système d'analyse d'au moins une zone à traiter,
ii. une source d'autres moyens d'activation choisi parmi une source de chaleur, de microondes, d'ondes sonores, une source lumineuse ne facilitant pas la réticulation du ou des composés,
iii. un système d'application d'une contrainte avant ou pendant l'illumination,
iv. un dispositif d'élimination des composés n'ayant pas réagi.

## Patentansprüche

1. Sterile Flüsssigzusammensetzung, die zur Verabreichung in oder über die Haut und/oder die Lippen ausgelegt ist, wobei die Zusammensetzung in einem physiologisch verträglichen Medium mindestens eine fotovernetzbare Verbindung umfasst, wobei die Verbindung mindestens eine fotodimerisierbare aktivierte Gruppe umfasst, die mindestens eine aktivierte Doppelbindung umfasst und die ausgewählt ist aus:
a) fotodimerisierbaren Gruppen, die eine Stilbazolium-Funktion der Formel (Ia) oder (1b) tragen: 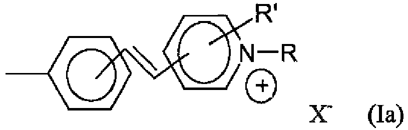 wobei
- R Wasserstoffatom, eine C₁-C₄-Alkylgruppe oder C₁-C₄-Hydroxyalkyl darstellt,
- R' Wasserstoffatom oder eine C₁-C₄-Alkylgruppe darstellt, und
- X⁻ ein Ion bezeichnet, das ausgewählt ist aus Chlor-, Brom-, lod-, Perchlorat-, Tetrafluorborat-, Methylsulfat-, Phosphat-, Sulfat-, Methansulfonat-, p-Toluolsulfonationen; 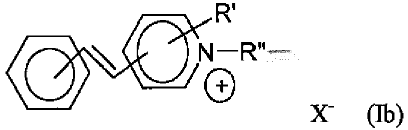
wobei
- R" einen zweiwertigen Alkylenrest mit 2 bis 8 Kohlenstoffatomen bezeichnet,
- R' Wasserstoffatom oder eine C₁-C₄-Alkylgruppe darstellt, und
- X⁻ die gleichen Bedeutung besitzt wie für die vorangehende Formel (Ia) beschrieben,
b) fotodimerisierbaren Gruppen, die eine Styrylazolium-Funktion der Formel (II) tragen: 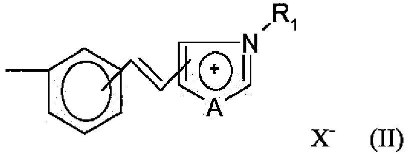 wobei:
- R¹ Wasserstoffatom, eine C₁-C₄-Alkylgruppe oder eine C₁-C₄-Hydroxyalkylgruppe bezeichnet,
- A ein Schwefelatom, ein Sauerstoffatom oder eine NR'- oder C(R')₂-Gruppe bezeichnet, wobei R', R' Wasserstoffatom oder eine C₁-C₄-Alkylgruppe bezeichnen, und
- X⁻ die gleichen Bedeutung besitzt wie für die vorangehende Formel (Ia) beschrieben,
wobei die fotodimerisierbare(n) Gruppe(n) von einem Polymer in der Art von teilweisem oder vollständig hydrolysierbarem Polyvinylacetat, einem Polysaccharid oder einem Polyvinylalkohol getragen werden.

2. Zusammensetzung nach Anspruch 1, **dadurch gekennzeichnet, dass** die fotodimerisierbare Gruppe eine fotodimerisierbare Gruppe ist, die eine Stilbazolium-Funktion der Formel (Ia) trägt: 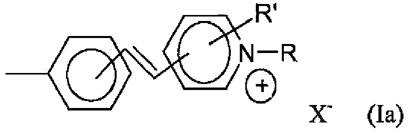 wobei
- R Wasserstoffatom, eine C₁-C₄-Alkylgruppe oder C₁-C₄-Hydroxyalkyl darstellt,
- R' Wasserstoffatom oder eine C₁-C₄-Alkylgruppe darstellt, und
- X⁻ ein Ion bezeichnet, das ausgewählt ist aus Chlor-, Brom-, lod-, Perchlorat-, Tetrafluorborat-, Methylsulfat-, Phosphat-, Sulfat-, Methansulfonat-, p-Toluolsulfonationen.

3. Zusammensetzung nach einem der vorangehenden Ansprüche,
**dadurch gekennzeichnet, dass** es frei ist von Fotostarter und/oder chemischem Starter.

4. Zusammensetzung nach einem der vorangehenden Ansprüche,
wobei die fotodimerisierbaren Gruppen von einem Polyvinylalkohol getragen werden.

5. Zusammensetzung nach einem der vorangehenden Ansprüche,
wobei die fotovernetzbare Verbindung ein Polyvinylalkohol ist, der zum Teil durch eine oder mehrere Hydroxylfunktion(en) und eine oder mehrere Funktion(en) der Formel (III) funktionalisiert ist:

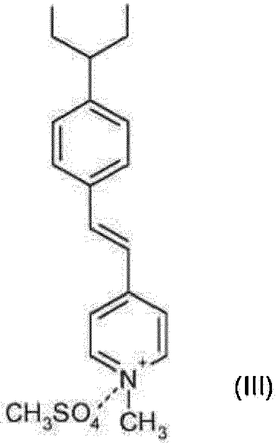

6. Zusammensetzung nach einem der vorangehenden Ansprüche,
**dadurch gekennzeichnet, dass** der Funktionalisierungsgrad mit fotodimerisierbarer Einheit mindestens 0,1 %, sogar mindestens 0,5 %, sogar mindestens 2 % beträgt.

7. Zusammensetzung nach einem der vorangehenden Ansprüche,
wobei die fotovernetzbare Verbindung mindestens 2 %, sogar mindestens 5 %, sogar 10 % des Gesamtvolumens der Zusammensetzung darstellt.

8. Zusammensetzung nach einem der vorangehenden Ansprüche,
weiterhin umfassend mindestens einen optischen Indikator oder eine fluoreszierende Verbindung.

9. Zusammensetzung nach einem der vorangehenden Ansprüche,
weiterhin umfassend mindestens einen zusätzlichen Wirkstoff, insbesondere einen Antifaltenwirkstoff.

10. Kosmetisches Behandlungsverfahren der Haut und/oder der Lippen,
umfassend die folgenden Schritte:
i. Verabreichung in oder durch die Haut und/oder die Lippen einer Zusammensetzung nach einem der Ansprüche 1 bis 9, und
ii. Bestrahlung der gemäß Schritt i behandelten Haut und/oder Lippen, um die Zusammensetzung zu vernetzen.

11. Kosmetisches Behandlungsverfahren der Haut und/oder der Lippen nach Anspruch 10, wobei die Bestrahlung eine Lichtquelle mit einer Strahlung im sichtbaren Bereich und/oder einer Strahlung im UV-Bereich und vorzugsweise einer Strahlung im sichtbaren Bereich ist.

12. Behandlungsverfahren der Haut und/oder der Lippen umfassend:
- ein Behältnis enthaltend mindestens eine Dosis einer Zusammensetzung nach einem der Ansprüche 1 bis 9 und,
- eine Injektionsvorrichtung in oder durch die Haut und/oder die Lippen oder eine zur Verabreichung der Dosis ausgelegte Mikroperforation der Haut und/oder der Lippen.

13. Behandlungsverfahren nach Anspruch 12, weiterhin umfassend einen Bildgeber.

14. Behandlungsverfahren nach einem der Ansprüche 12 und 13, weiterhin umfassend eine Injektionsvorrichtung, die zur intraepidermalen und/oder intradermalen und/oder subkutanen Injektion geeignet ist.

15. Behandlungsverfahren nach einem der Ansprüche 12 bis 14, wobei die Injektionsvorrichtung ausgewählt ist aus einer Spritze, einer Gesamtheit von Mikrospritzen, einer Laser-, Hydraulikvorrichtung oder einem Skalpell.

16. Behandlungsverfahren nach einem der Ansprüche 12 bis 14, wobei die Injektionsvorrichtung zur Mesotherapietechnik geeignet ist.

17. Behandlungsgesamtheit der ästhetischen Unzulänglichkeiten der Haut und/oder der Lippen umfassend:
i. ein Behandlungssystem nach einem der Ansprüche 12 bis 16, und
ii. ein Beleuchtungssystem, das zum Fotovernetzen der Verbindung ausgelegt ist.

18. Behandlungsgesamtheit nach Anspruch 17, weiterhin umfassend mindestens:
i. ein Analysesystem von mindestens einem zu behandelndem Bereich,
ii. eine Quelle für andere Aktivierungsmittel, die ausgewählt ist aus einer Wärmequelle, einer Mikrowellenquelle, einer Schallquelle, einer Lichtquelle, die die Vernetzung der Verbindung oder der Verbindungen nicht erleichtert,
iii. ein Applikationssystem eines abgegrenzten Bereichs vor oder während der Bestrahlung,
iv. eine Beseitigungsvorrichtung der Verbindungen, die noch nicht reagiert haben.

## Claims

1. A sterile liquid composition dedicated to administration into or through the skin and/or lips, said composition in a physiologically acceptable medium comprising at least one photo-crosslinkable compound, said compound comprising at least one activated photodimerizable group having at least one activated double bond and selected from among:
a) photodimerizable groups carrying a stylbazolium function of formula (Ia) or (Ib): 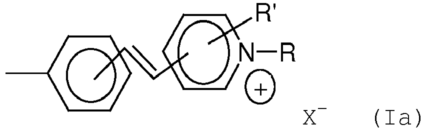 where:
- R is the hydrogen atom, C₁ to C₄ alkyl or C₁ to C₄ hydroxyalkyl group;
- R' is the hydrogen atom or C₁ to C₄ alkyl group; and
- X⁻ denotes an ion selected from among chloride, bromide, iodide, perchlorate, tetrafluoroborate, methylsulfate, phosphate, sulfate, methanesulfonate and p-toluenesulfonate ions. 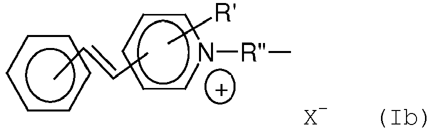 where:
- R" denotes a divalent alkylene radical having 2 to 8 carbon atoms;
- R' is the hydrogen atom or C₁ to C₄ alkyl group; and
- X⁻ having the same meaning as described for preceding formula (Ia).
b) the photodimerizable groups carrying a styrylazolium function of formula (II): 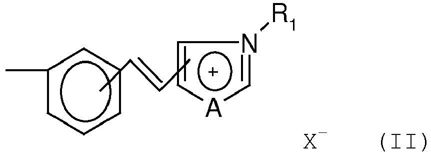 wherein:
- R₁ is the hydrogen atom, C₁ to C₄ alkyl or C₁ to C₄ hydroxyalkyl group;
- A denotes a sulfur atom, an oxygen atom, NR' or C(R')₂, group, with R', R' representing the hydrogen atom or C₁ to C₄ alkyl group; and
- X⁻ having the same meaning as described for preceding formula (Ia),
the photodimerizable group(s) being carried by a polymer of partly or fully hydrolysed polyvinylacetate type, a polysaccharide or polyvinyl alcohol.

2. The composition according to claim 1, **characterized in that** the photodimerizable group is a photodimerizable group carrying a stylbazolium function of formula (Ia) 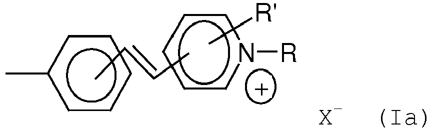 where:
- R is the hydrogen atom, C₁ to C₄ alkyl or C₁ to C₄ hydroxyalkyl group;
- R' is the hydrogen atom or C₁ to C₄ alkyl group; and
- X⁻ denotes an ion selected from among chloride, bromide, iodide, perchlorate, tetrafluoroborate, methylsulfate, phosphate, sulfate, methanesulfonate, p-toluenesolfonate ions.

3. The composition according to any of the preceding claims, **characterized in that** it is devoid degree of photoinitiator and/or chemical initiator.

4. The composition according to any of the preceding claims, wherein the photodimerizable groups are carried by a polyvinyl alcohol.

5. The composition according to any of the preceding claims, wherein the photo-crosslinkable compound is a polyvinyl alcohol partly functionalised by one or more hydroxyl functions and one or more functions of formula (III):

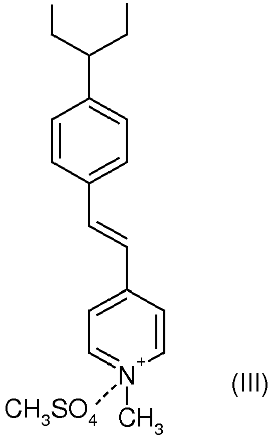

6. The composition according to any of the preceding claims, **characterized in that** the degree of functionalisation to photodimerizable repeating unit is at least 0.1 %, even at least 0.5 %, even at least 2 %.

7. The composition according to any of the preceding claims, wherein the photo-crosslinkable compound represents at least 2 %, even at least 5 %, even 10 % of the total volume of the composition.

8. The composition according to any of the preceding claims, further comprising at least one optical tracer or fluorescent compound.

9. The composition according to any of the preceding claims, further comprising at least one additional active ingredient, in particular an anti-wrinkle active ingredient.

10. A cosmetic method to treat the skin and/or lips comprising the following steps:
i) administering into or through the skin and/or lips a composition according to any of claims 1 to 9; and
ii) illuminating the skin and/or lips treated at step i) to crosslink the composition.

11. The cosmetic method to treat the skin and/or lips according to claim 10, wherein the illumination is a light source with radiation in the visible range and/or radiation in the UV range, and preferably radiation in the visible range.

12. A system to treat the skin and/or lips, comprising:
- packaging containing at least one dose of a composition according to any of claims 1 to 9; and
- an injection device into or through the skin and/or lips, or microperforation device of the skin and/or lips dedicated to administering said dose.

13. The treatment system according to claim 12 further comprising an imager.

14. The treatment system according to any of claims 12 and 13 further comprising an injection device adapted for intraepidermnal and/or intradermal and/or subcutaneous injection.

15. The treatment system according to any of claims 12 to 14, wherein the injection device is selected from among a syringe, set of microsyringes, hydraulic laser device or scalpel.

16. The treatment system according to any of claims 12 to 14, wherein the injection device is suitable for the mesotherapy technique.

17. An assembly for the treatment of cosmetic imperfections of the skin and/or lips, comprising.
i) a treatment system according to any of claims 12 to 16; and
ii) an illuminating system capable of causing crosslinking of the compound.

18. A treatment assembly according to claim 17 further comprising at least:
i) a system to analyse at least one area to be treated;
ii) a source of other activation means selected from among heat, microwave, soundwave sources, a light source not facilitating crosslinking of the compound(s);
iii) a system to apply stress before or during illumination;
iv) a device to remove the compounds that have not reacted.
